# EUROPÄISCHE PATENTSCHRIFT

(11) **EP 1 412 323 B1**
(45) Veröffentlichungstag und Bekanntmachung des Hinweises auf die Patenterteilung: **30.09.2009**
(21) Anmeldenummer: 02794553.4
(22) Anmeldetag: 05.08.2002
(51) Int. Cl.: C07C 255/13, C07C 255/12, C07C 255/20, C07C 253/30, C07D 319/06

(54) **GESCHÜTZTE 3,5-DIHYDROXY-2,2-DIMETHYL-VALERONITRILE FÜR DIE SYNTHESE VON EPOTHILONEN- UND DERIVATEN UND VERFAHREN ZUR HERSTELLUNG UND DIE VERWENDUNG**
PROTECTED 3,5-DIHYDROXY-2,2-DIMETHYL-VALERONITRILES FOR THE SYNTHESIS OF EPOTHILONES AND DERIVATIVES AND METHOD FOR THE PRODUCTION AND USE THEREOF
3,5-DIHYDROXY-2,2-DIMETHYL-VALERONITRILES PROTEGES DESTINES A LA SYNTHESE D'EPOTHILONES ET DE DERIVES DE CELLES-CI, PROCEDES DE PRODUCTION DE CES COMPOSES ET UTILISATION DE CEUX-CI

(30) Priorität: 03.08.2001 DE 10138347
(43) Veröffentlichungstag der Anmeldung: 28.04.2004
(73) Patentinhaber: Bayer Schering Pharma Aktiengesellschaft, 13353 Berlin (DE)
(72) Erfinder: WESTERMANN, Jürgen, 12161 Berlin (DE); PETROV, Orlin, 14199 Berlin (DE); PLATZEK, Johannes, 12621 Berlin (DE)
(86) Internationale Anmeldenummer: PCT/EP2002/008730
(87) Internationale Veröffentlichungsnummer: WO 2003/014068

(56) Entgegenhaltungen:
- WO-A-00/58254
- SCHINZER D ET AL: "CHEMISTRY - A EUROPEAN JOURNAL, VCH PUBLISHERS, US" CHEMISTRY - A EUROPEAN JOURNAL, VCH PUBLISHERS, US, Bd. 2, Nr. 11, 1996, Seiten 1477-1482, XP002095724 ISSN: 0947-6539
- SCHINZER D ET AL: "ANGEWANDTE CHEMIE, VCH VERLAGSGESELLSCHAFT, WEINHEIM, DE" ANGEWANDTE CHEMIE, VCH VERLAGSGESELLSCHAFT, WEINHEIM, DE, Bd. 109, Nr. 5, 1997, Seiten 543-544, XP002095720 ISSN: 0044-8249
- JOHANN MULZER ET AL: "Total Syntheses of Epothilones B and D" J. ORG. CHEM., Bd. 65, Nr. 22, 2000, Seiten 7456-7467, XP002218886 in der Anmeldung erwähnt

## Beschreibung

Die Erfindung betrifft den in den Patentansprüchen gekennzeichneten Gegenstand, das heißt neue Zwischenprodukte und Verfahren zu deren Herstellung und die Verwendung. Das Verfahren zur Herstellung neuer Zwischenprodukte geht von kostengünstigen Ausgangsmaterialien aus, liefert die Zwischenprodukte in hohen Enantiomerenreinheiten, in hoher chemischer Reinheit, in guten Ausbeuten und erlaubt die großtechnische Herstellung.

Die Erfindung wird bei der Synthese des Bausteins A von natürlichen und synthetisch modifizierten Epothilonen oder Derivaten verwendet. Epothilone sind 16 gliedrige Macrolidringe, die aus Kulturen des Myxobacteriums Sorangium Cellosum isoliert wurden und sind Vertreter einer Klasse von vielversprechenden Antitumormitteln, die als wirksam gegen eine Reihe von Krebslinien getestet wurden. Eine Übersicht zu den Synthesen ist von J. Mulzer et al. in J. Org. Chem. 2000, 65,7456-7467 beschrieben worden.

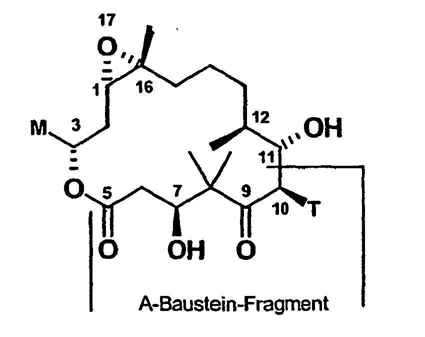

In der Literatur sind neben den natürlichen Epothilonen eine Vielzahl synthetischer Epothilonderivate beschrieben, die zum größten Teil innerhalb der Reste M und T variieren. M steht hier meistens für einen heterocyclischen Rest. Die meisten Synthesen der natürlichen Epothilone und der Synthetischen Epothilonderivate benutzen das A-Baustein-Fragment, welches die Kohlenstofftome C₅ - C₁₀ im Macrolid darstellen. Innerhalb dieses Bausteines A (s.u.) ist C₁ das C₅ im Macrolid und C₆ das C₁₀ im Macrolid, usw.

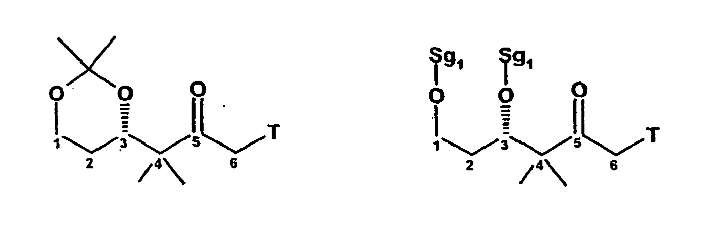

Hierbei steht **T** für einen C1-C4 Alkyl oder Alkenyl-Rest, Sg1 und Sg2 stehen für dem Fachmann geläufige Schutzgruppen, wie z.B. die TBDMS-Gruppe.

Eine mögliche Herstellung des A-Bausteins wird beispielsweise in der WO00/58254 beschrieben. Darin wird eine Synthese aus β-Ketoestern offenbart, die in mehrstufigen Sequenzen in den Baustein A überführt werden können. Die Chiralität wird durch eine asymmetrische Hydrierung eines β-Ketoesters nach Noyori eingeführt :

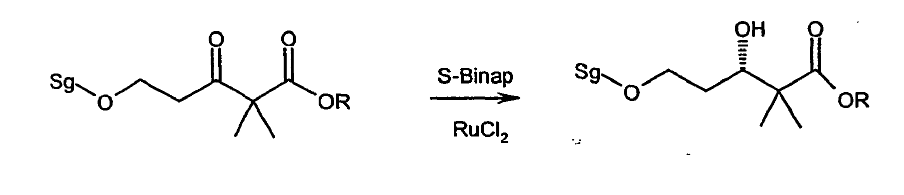

Die Umwandlung der Estergruppe in ein Keton ist hierbei nur mittels einer mehrstufigen Sequenz realisierbar. Dabei wird nach einem Schutz der 1 und 3-Hydroxygruppe die Estergruppe (C-5 Atom) zum Alkohol reduziert, die Oxidation zum Aldehyd erfolgt, die Grignardaddition eines Alkylrestes mit einer Alkylmagnesium bzw. Alkyllithiumverbindung liefert einen sekundären Alkohol, der anschließend oxidiert wird. Um von dem Ester zum Keton zu gelangen, sind insgesamt 8 Schritte erforderlich. Die direkte Umsetzung eines Esters ist nicht selektiv, da das intermediär hergestellte Produkt weiterreagiert. Das folgende Schema zeigt den gesamten Syntheseweg :

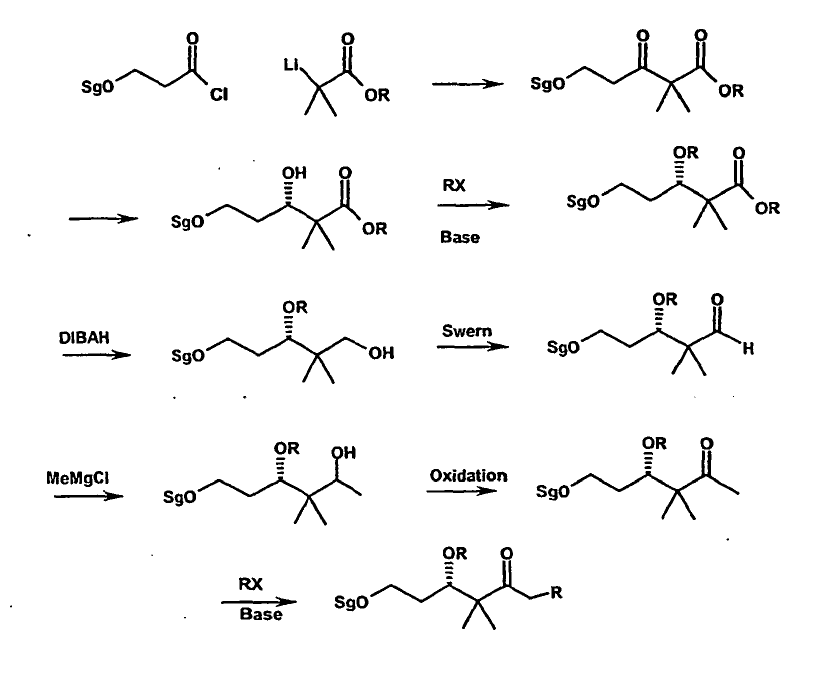

Von B. Paniker et al. wird in Tetrahedron 2000, 56,78-59-7868 eine Methode zum Aufbau des Bausteins A beschrieben. Dort wird beschrieben, dass die Aldolreaktion mit einem chiralen Baustein eine wenig selektive Reaktion liefert. Ober den Umweg eines N-Methylthioacetyl-oxazolidinons wird die Synthese des chiralen C3-Atoms in einer mehrstufigen Sequenz mit verbesserter Diastereoselektivität mittels einem Borenolat beschrieben. Zur Erzielung brauchbarer Diastereoselektivitäten ist eine Methylthiosubstitution erforderlich; der Thioether wird nach der Aldolreaktioh abgespalten.

Weiter ist dem Stand der Technik (R. E. Taylor, Y. Chen, Org. Lett. (2001), 3(14), 2221-2224) eine Sequenz zu entnehmen, bei der ein Phenylester für die Grignard-Reaktion verwendet wird. Die dabei erzielte Ausbeute wird mit 77 % angegeben. In dem von A. Fürstner, in Chem. Comm.2001, 1057-1059 beschriebenen Beispiel werden 67 % Ausbeute erreicht. Diese Ausbeuten der Grignard-Reaktion aus dem Stand der Technik sind deutlich niedriger als diese der vorliegenden Erfindung.

In J. Org. Chem. 2000, 65, 7456-7467 wird weiter eine asymmetrische Synthese eines β-Ketoesters beschrieben, wobei eine Variante in asymmetrischer Form als Aldolreaktion durchgeführt wird. Als Katalysator wird bei dieser Methode D-Ts-Valin verwendet, das sich aus der teuren Aminosäure D-Valin herstellen lässt. Diese Methode liefert einen ee-Wert von 90%. Ein weiteres Beispiel dazu wird von R. E. Taylor, Y. Chen, Org. Lett. (2001), 3(14), 2221-2224 als eine asymmetrische Aldolreaktion beschrieben, bei der die Ausbeute 71 % beträgt.

Eine weitere Methode zur Herstellung eines doppelt TBDMS-geschützten A-Baustein- Ethylketons wird schließlich von Nicolaou in Chem. Eur. J. 2000, 6, 2783-2800 beschrieben.

Die vorliegende Erfindung beinhaltet die Aufgabe ein universell einsetzbares Ausgangs-Intermediat der allgemeinen Formel I sowie der optisch reinen Antipoden der allgemeinen Formeln Ia, Ib herstellen zu können. 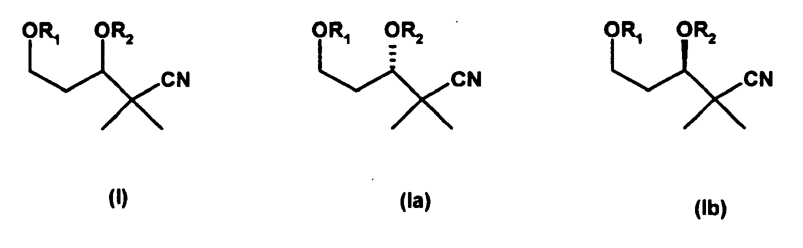 worin R1, R2 gleich oder verschieden sein können und unabhängig voneinander für eine dem Fachmann geläufige Alkoholschutzgruppe beispielsweise Benzyl, 4-Methoxybenzyl , 3,4-Dimethoxybenzyl ,THP, TBDMS, TMS, TES, TIP, TBDPS, MEM , MOM, Allyl, Trityl,
oder im Falle wenn R1 und R2 verbrückt sind für eine Ketalschutzgruppe wie z.B. 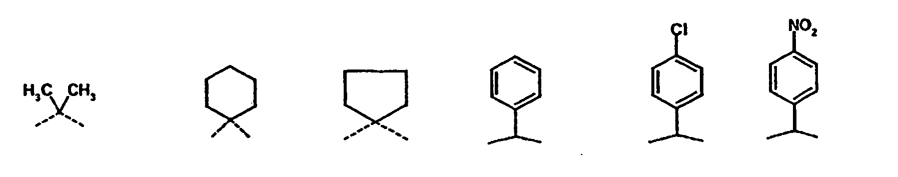 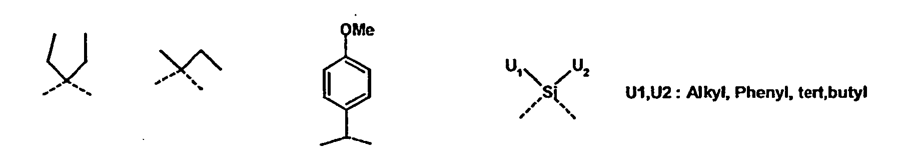 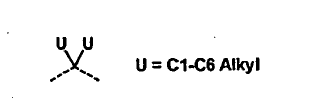 stehen,
um A-Baustein-Fragmente für Epothilon-Totalsynthesen herzustellen.

Hierzu setzt man Verbindungen der allgemeinen Formel I wie nachfolgend beschrieben um :

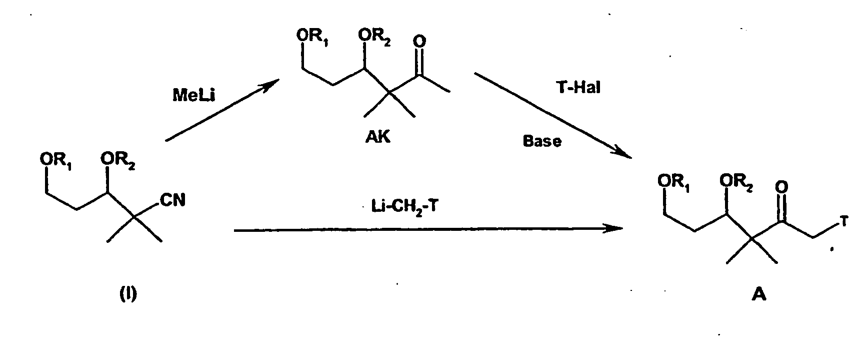

Die Umsetzungen von Verbindungen der allgemeinen Formel I, sowie deren Antipoden Ia,Ib zu den Ketonen AK erfolgen mit Methyllithium oder Methylgrignard-Verbindungen nach dem Fachmann bekannten Standardverfahren, die wässrige Aufarbeitung liefert dann das Keton. Die anschließende Alkylierung mit einem Alkyl oder Alkenyl-Halogenid der Formel T-Hal (Hal = Cl, Br, J, oder Tosylat, Mesylat, Triflat etc.) unter Zusatz einer Base liefert die A-Bausteinfragmente.

Man kann aber auch direkt zu A gelangen, indem man die Amide der allgemeinen Formel I direkt mit metallorganischen Verbindungen wie z.B. der Lithium-Verbindung Li-CH2-T umsetzt und anschließend wässrig aufarbeitet.
Die oben beschriebenen Umsetzungen laufen in der Regel glatt und liefern die A-Bausteine in hohen Ausbeuten.
Es bestand daher ein Bedarf nach einem großtechnischen Verfahren, das es erlaubt, ein universell einsetzbares Intermediate für die Herstellung des A-Bausteins bei der Epothilon-Totalsynthese bereitzustellen.
Neben den hohen Ausbeuten bei der Überführung in die A-Bausteine ist die relativ leichte Zugänglichkeit der Verbindungen der allgemeinen Formel I aus relativ billigen Ausgangsmaterialien hervorzuheben . Darüber hinaus sind die erfindungsgemäßen Verbindungen im Gegensatz zu den literaturbekannten Estern und Ketonen lagerstabil und können während einer laufenden Synthesekampagne nach Bedarf umgesetzt werden. Die Verbindungen der allgemeinen Formel I sind zum größten Teil kristalline Feststoffe und lassen sich durch Kristallisation reinigen. Auf diese Weise lassen sich hohe chemische und optische Ausbeuten (e.e.> 98 %) erzielen.

Die Aufgabe der Erfindung wird durch die Bereitstellung der neuen Verbindungen der allgemeinen Formel I, Ia,Ib 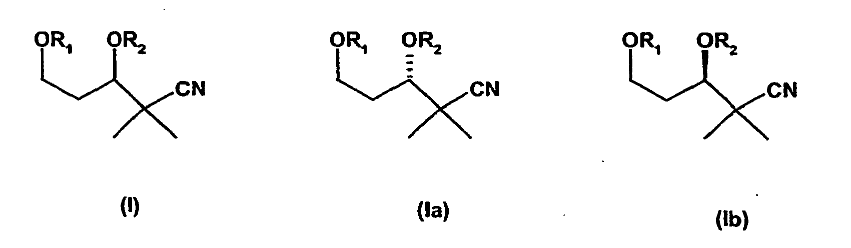 gelöst,
worin R1, R2 gleich oder verschieden sein können und unabhängig voneinander für eine Alkoholschutzgruppe wie z.B. Benzyl, 4-Methoxybenzyl 3,4-Dimethoxybenzyl THP, TBDMS, TMS, TES, TIP, TBDPS, MEM , MOM, Allyl, Trityl,
oder im Falle wenn R1 und R2 verbrückt sind für eine Ketalschutzgruppe wie z.B. 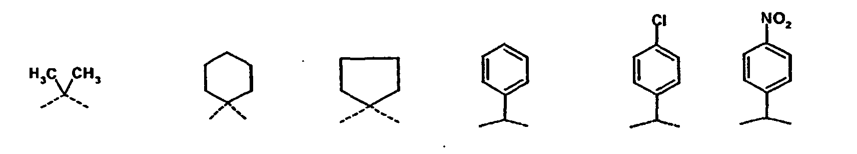 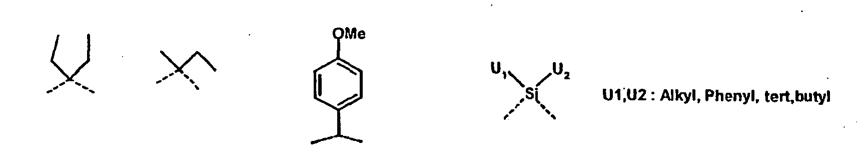 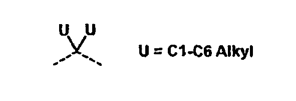 stehen.

Zur Herstellung der erfindungsgemäßen Verbindungen lassen sich insgesamt **4** Varianten angeben:

### Variante I (allgemeiner Zugang über Aldolreaktionen)

a) In dem Fall, wo R1 und R2 für eine Ketal-Schutzgruppe stehen, oder R1 = R2 lassen sich Verbindungen der allgemeinen Formel I aus Verbindungen der Formel II, 2,2-Dimethyl-3,5- 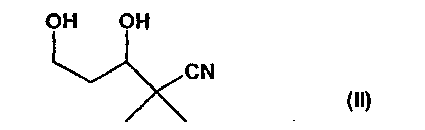 dihydroxy-valero-nitril,
   nach den dem Fachmann bekannten Methoden zur Schutzgruppenchemie herstellen, so ist beispielsweise deren Herstellung und Abspaltung bei P.J. Kocienski in "Protecting Groups", Georg Thieme Verlag Stuttgart, New York 1994 beschrieben, sowie in Houben Weyl, 4th. Ed. Vol VI/1b p. 737, Thieme Stuttgart 1984 beschrieben.
b) Im Falle, dass R1 und R2 keine Ketal-Schutzgruppe darstellen aber dennoch gleich oder verschieden sein können, kann die Herstellung von Verbindungen der allgemeinen Formel I direkt aus Verbindungen der allgemeinen Formel III erfolgen, indem man die Schutzgruppe R2 nach literaturbekannten Methoden einführt.

Verbindungen der allgemeinen Formel II lassen sich aus Verbindungen der allgemeinen Formel III 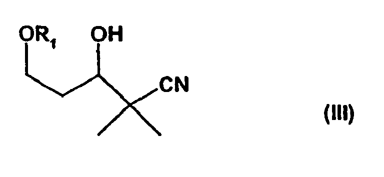 worin R1 für eine Schutzgruppe in der oben angegebenen Bedeutung steht, durch Abspaltung der Schutzgruppe R1 nach dem dem Fachmann bekannten Verfahren der Schutzgruppen-Abspaltung von Alkoholen herstellen (P.J. Kocienski in "Protecting Groups", Georg Thieme Verlag Stuttgart, New York 1994/ Houben Weyl, 4th. Ed. Vol VI/1b p. 737, Thieme Stuttgart 1984) herstellen.

Verbindungen der allgemeinen Formel III lassen sich aus Verbindungen der allgemeinen Formel IV 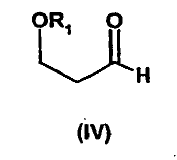 durch Umsetzung mit der Verbindung der Formel V, 2-Methylproionitril, 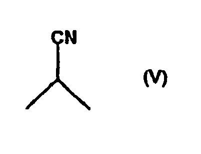 in der R1 in der oben angegebenen Bedeutung steht,
in an sich dem Fachmann bekannter Weise durch die Techniken der Aldolkondensation herstellen.

Die Herstellung von Verbindungen der allgemeinen Formel IV sind aber dem Fachmann bekannt:
R1 = THP in JOC, 1984, 49, 2301-2309
R1 = Benzyl in J. Chem. Soc. Perk. Trans 1, 2000, 2429-2454.
R1 = TBDMS in JOC, 2000, 65, 7456-7467

Die Verbindung Formel V, 2-Methylpropionitril, ist Kaufware.

### Variante II (Herstellung optisch aktiver Zwischenprodukte der allgemeinen Formel Ia)

Zur Herstellung von optisch aktiven Verbindungen der allgemeinen Formel Ia 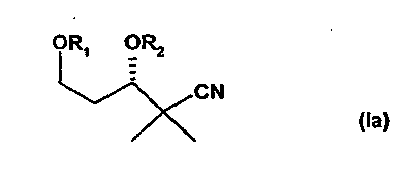 wird in analoger Weise wie unter Variante I beschrieben verfahren. Ausgehend von der optisch aktiven Zwischenstufe der allgemeine Formel IIa und IIIa 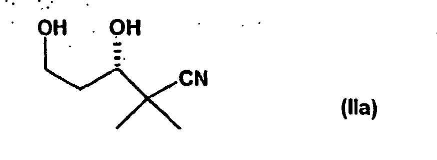 werden Verbindungen der allgemeinen Formel Ia hergestellt.

Verbindungen der allgemeinen Formel IIa werden in analoger Weise aus der optisch aktiven Vorstufe der allgemeinen Formel IIIa 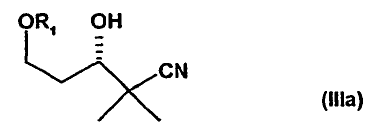 hergestellt.

Optisch aktive Verbindungen der allgemeinen Formel IIIa sind wie folgt zugänglich :
1. Trennung der racemischen Verbindung der allgemeinen Formel III an chiraler Phase (Lit.: G. Roussel, P. Piras, Chirabase, Pure and Applied Chemistry, 1993, 65, 235-244), vor allen durch SMB-Technik: A. Seidel-Morgenstern et al. , Chromat. A. 1998, 827/2, 175-191.
2. indem man ausgehend vom racemischen Alkohol der allgemeinen Formel III Ester der allgemeinen Formel VI 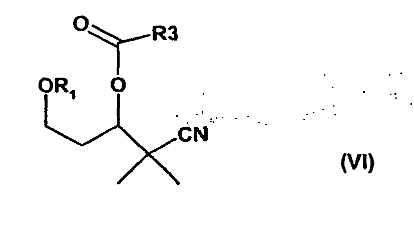 worin R3 für ein C1-C6 Alkylgruppe oder ein Alyll- -Phenyl-, oder Benzylgruppe steht, nach dem Fachmann bekannten Verfahren der Veresterung herstellt.
   Und diese durch enzymatisch bzw. mikrobiologische Methoden enantioselektiv verseift. Der entstehende Alkohol unterscheidet sich in seinem Rf-Wert deutlich vom eingesetzten Ester, sodass beide bequem voneinander getrennt werden können, z.B. durch Säulenchromatographie.
3. Durch eine mit chiralen Katalysatoren vermittelte Aldolkondensation indem man Verbindungen der allgemeinen Formel IV und V unter Verwendung von katalytischer, bzw. stöchiometrischer Menge eines chiralen Aldolkatalysators umsetzt: 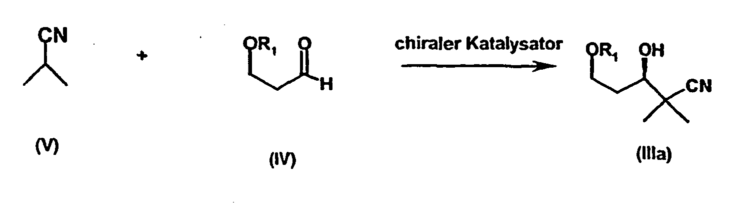 Literatur: siehe z.B. J. Org. Chem. 2000, 65, 7456-7467
4. Indem man eine chirale Reduktion des Ketons der allgemeine Formel VII 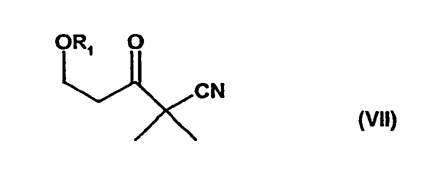 nach dem Fachmann bekannten Methoden durchführt. Lit. : Noyori et al., J.Am. Chem. Soc. 1987, 109, 5850; Noyori et al., J. Am. Chem. Soc. 1988, 110, 629, R.C. Larock in "Comprehensive Organic Transformations". VCH Publishers New York 1989, ISBN 0-89573-710-8, Seite 540-548.

Verbindungen der allgemeinen Formel VII, mit R1 in der oben angegebenen Bedeutung lassen sich durch Umsetzung von Verbindung der Formel V mit Verbindungen der allgemeinen Formel VIII erhalten 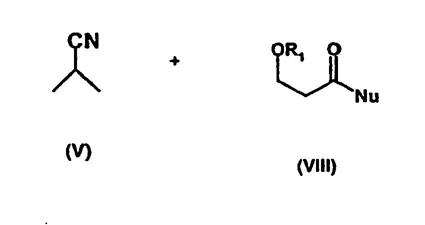 worin Nu für eine Fluchtgruppe wie Cl, Br, Imidazol, -OPh, -O-C6H4NO2, -O-C1-C4 Alkyl etc. steht.
Die Umsetzung erfolgt in an sich dem Fachmann bekannter Weise.

Die Herstellung von Verbindungen der allgemeinen Formel VIII ist in der Literatur beschrieben : J.Med.Chem. 1999, 706-721.

In einigen Fällen hat es sich als günstig erwiesen, wenn man Verbindungen der allgemeinen Formel VII durch Oxidation aus den racemischen Alkohole der allgemeinen Formel II nach dem dem Fachmann bekannten Methoden der Oxidation herstellt (z.B. Swern-Oxidation, PDC, PCC, etc.)

In einigen Fällen hat es sich als günstig erwiesen, wenn man Verbindung der Formel V mit Propiolacton zur Verbindung der IX umsetzt

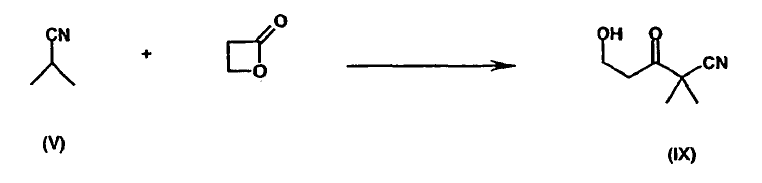

Die Verbindung der Formel IX kann sehr leicht durch Einführung von Schutzgruppen nach den dem Fachmann bekannten Methoden in Verbindungen der allgemeinen Formel VII überführt werden (siehe : P.J. Kocienski in "Protecting Groups", Georg Thieme Verlag Stuttgart, New York 1994 beschrieben, sowie in Houben Weyl, 4th, Ed. Vol VI/1b p. 737, Thieme Stuttgart 1984).

Man kann aber jedoch auch, ausgehend von Verbindungen der Formel IX zu Verbindung der Formel IIa 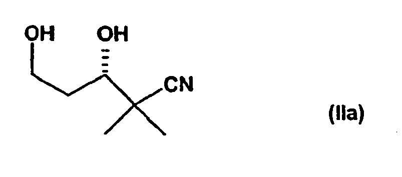 gelangen, indem man die Ketogruppe mit chemischen oder mikrobiologischen Methoden chiral reduziert (z.B. nach : JOC1985, 50, 127 / J.Chem. Soc., Chem. Commum. 1987, 1368).

### Variante III

Verbindungen der allgemeinen Formel Ia 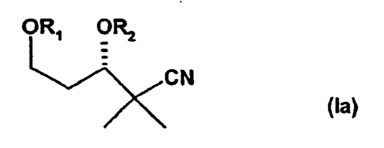 können auch durch Einführung von Schutzgruppen nach literaturbekannten Methoden zur Einführung von Alkoholschutzgruppen aus den Verbindungen allgemeinen Formel X 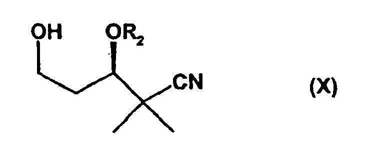 hergestellt werden (siehe oben angeführte Literatur zu Einführung von Schutzgruppen). Verbindungen der allgemeinen Formel X können aus Verbindungen der allgemeinen Formel XI 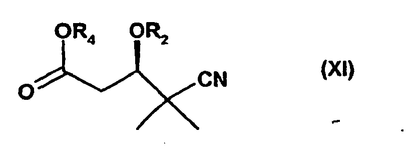 worin R4 für eine Methyl, Ethyl, Benzylgruppe steht,
durch Esterreduktion nach dem Fachmann bekannten Methoden hergestellt werden.

Verbindungen der allgemeinen Formel XI können aus Verbindungen der allgemeinen Formel XII 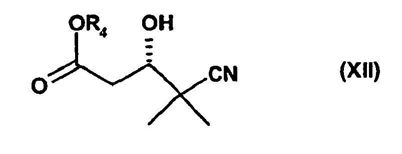 worin R4 für eine C1-C6 Alkyl, Methyl, Ethyl, tert.Butyl, Phenyl oder Benzylgruppe steht, Durch Einführung der Schutzgruppe R2 nach dem Fachmann bekannten Methoden hergestellt werden (siehe oben).

Verbindungen der allgemein Formel XII lassen sich aus β-Ketoestern der allgemeinen 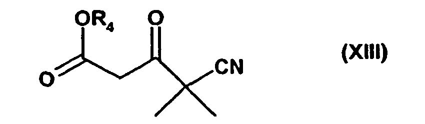 Formel XIII
durch Methoden der chiralen Reduktion, (chemisch oder enzymatisch) erhalten.

Verbindungen der allgemeinen Formel XIII werden durch Umsetzung aus Verbindungen der allgemeinen Formel XIV mit Verbindung der Formel V erhalten 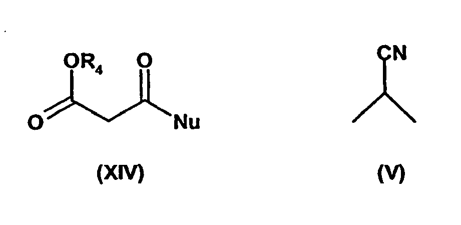 Verbindungen der allgemeinen Formel XIV sind literaturbekannt
oder können auch aus der Umsetzung von Verbindungen der allgemeinen Formel Xllla und XIII b erhalten werden

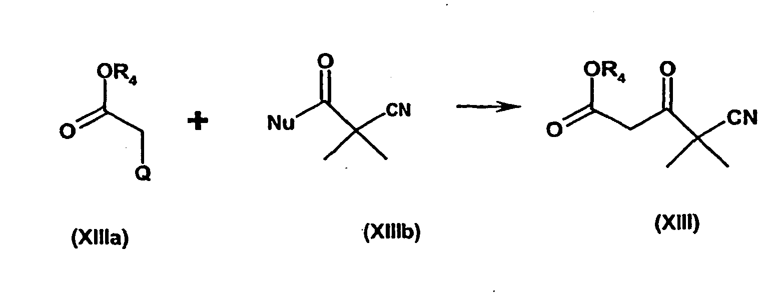

Hierbei steht Nu in der Bedeutung der bereits oben erwähnten Fluchtgruppe, Q steht für ein Wasserstoffatom oder eine COOH-Gruppe. Falls Q ein Wasserstoffatom ist, wird XIIIa mit einer organischen Base wie z.B. LDA deprotoniert und anschließend mit dem aktivierten Säurederivat nach dem Fachmann geläufigen Methoden umgesetzt.

Im Falle von Q gleich COOH wird mit den Methoden der Malonsäure-Halbester Kondensation verfahren, wie z.B. in J. Am. Chem. Soc. 1999, 121, 7050-7062, Synth. Commun. 1997, 27, 3227-3234 beschrieben.

Verbindungen der allgemeinen Formel XIIIa sind käuflich erhältlich (z.B. Aldrich).

Verbindungen der allgemeinen Formel Xlllb werden wie in R.C. Larock in "Comprehensive Organic Transformations", VCH Publishers New York 1989, ISBN 0-89573-710-8, Seite 963-964, beschrieben hergestellt.

In einigen Fällen hat es sich als günstig erwiesen, die Diole der allgemeinen Formel IIa 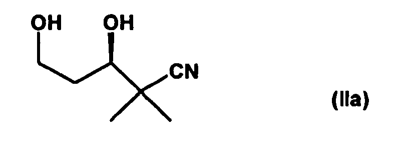 direkt aus den Verbindungen der allgemeinen Formel XII 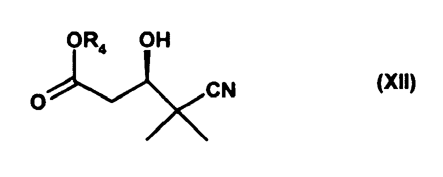 durch Reduktion der Estergruppe, nach den oben genannten Verfahren durchzuführen.

Zur Herstellung von racemischen Diol der allgemeinen Formel II kann auch ausgehend von β-Ketoestern der allgemeinen Formel XIII 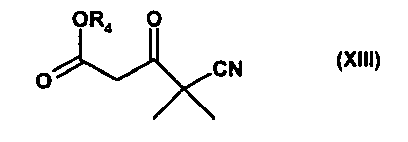 nach den üblichen Methoden zur Reduktion von Estern und Ketonen ausgegangen werden.

### Variante IV

In einigen Fällen hat es sich als günstig erwiesen, dass man zur Herstellung der optisch aktiven Diole der allgemeinen Formel IIa eine chromatographische Trennung oder Kristallisation der diastereomeren Ketale der allgemeinen Formeln XIVa und XIVb 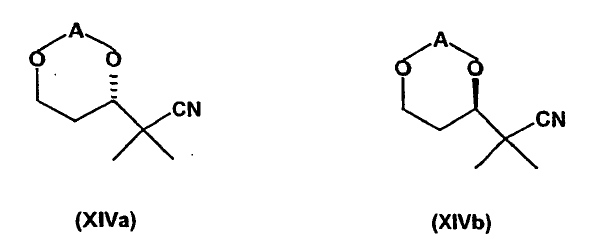 worin A für den Rest eines optisch aktiven Ketons, wie z.B. (-) Menthon, (-) Kampfer etc. vornimmt, und anschließend die Ketalgruppe nach den dem Fachmann bekannten Methoden der Schutzgruppenchemie abspaltet.

Die Herstellung von diastereomeren 1,3 Diol-Ketalen der allgemeinen Formel XIVa und XIVb erfolgt aus dem racemischen Diol der allgemeinen Form II durch Umsetzung mit chiralen Ketonen nach literaturbekannten Verfahren. Lit.: T. Harada et al. , J. Org. Chem. 1992, 57, 1412 - 142-1.

Natürlich können auch unter Verwendung spiegelbildlicher Katalysatoren, bzw. anderer Enzymsysteme die entsprechenden enantiomeren Verbindungen der allgemeinen Formel Ib hergestellt werden

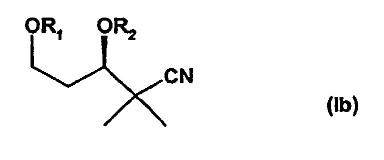

Es besteht ebenfalls die Möglichkeit bei Zwischen-Stufen der allgemeinen Formel IIIb 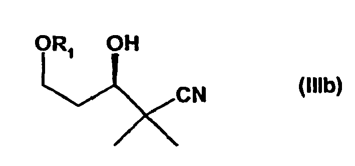 durch Inversion der Hydroxylgruppe nach Mitsunobu (Lit. : Synthesis 1981, 1 - 28) zu den entsprechenden Enantiomeren zu gelangen.

Von denen in der Synthese verwendeten Schutzgruppen R1 und R2 sind die Benzylgruppe, die TBDMS-Gruppe bevorzugt. Im Falle , dass R1, R2 für eine Ketalschutzgruppe steht, ist besonders -(C(CH3)2)- bevorzugt. '

Von den hier verschiedenen Varianten der Herstellung sind besonders die folgenden Teil-Sequenzen zum Aufbau der achiralen Vorstufen bevorzugt:
1. Herstellung von Verbindung der allgemeinen Formel VII mit aus Zwischenstufen der allgemeinen Formel. V und VIII
   R1 = Benzyl, Nu = Cl 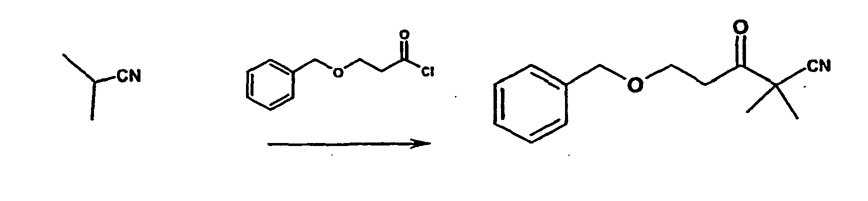
2. Herstellung von Verbindung der allgemeinen Formel XIII aus Verbindungen der allgemeinen Formel V und XIV
   R4 = Ethyl , Nu = Cl 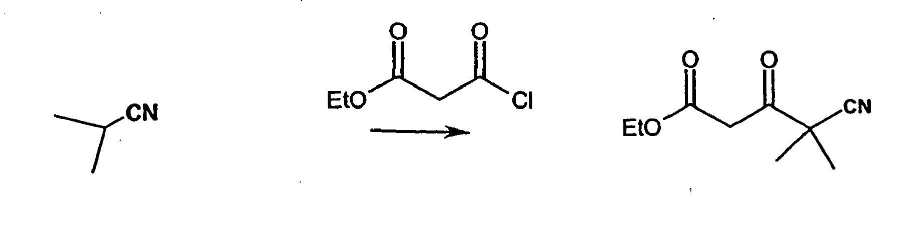
3. Herstellung von Verbindungen der allgemeinen Formel VII durch Aldolkondensation und nachfolgender Oxidation
   R1 = Benzyl, Nu = Cl 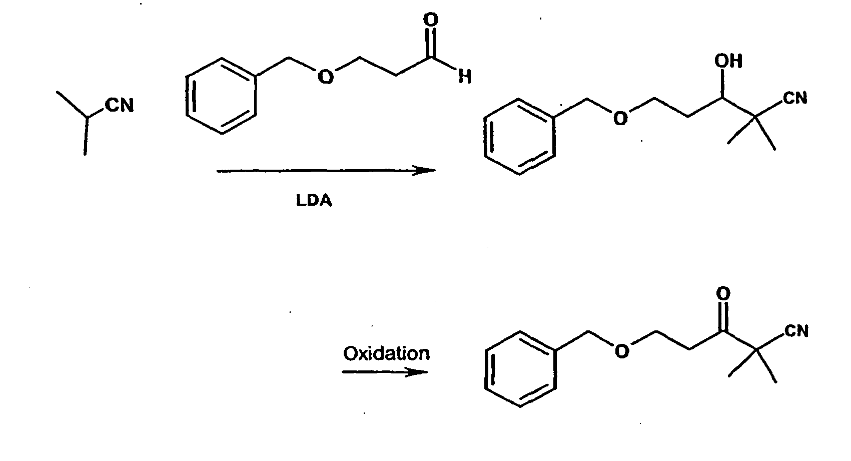
4. Herstellung von Verbindungen der allgemeinen Formel IX (mit Y = Dimethylamino)

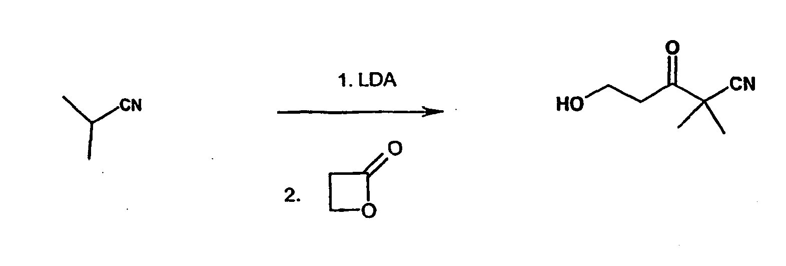

Zur Herstellung chiraler Vorstufen sind besonders die nachfolgend angegebenen Teilschritte bevorzugt:
1. Chirale Aldolkondensation mit chiralem Katalysator 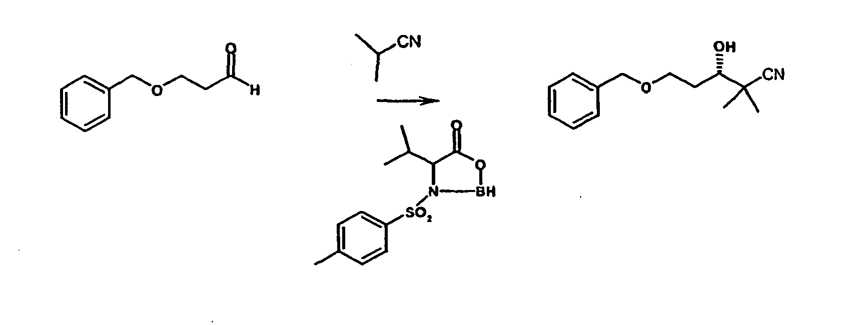
2. Enantioselektive Verseifung von einem Acetat mit Hilfe eines Enzyms 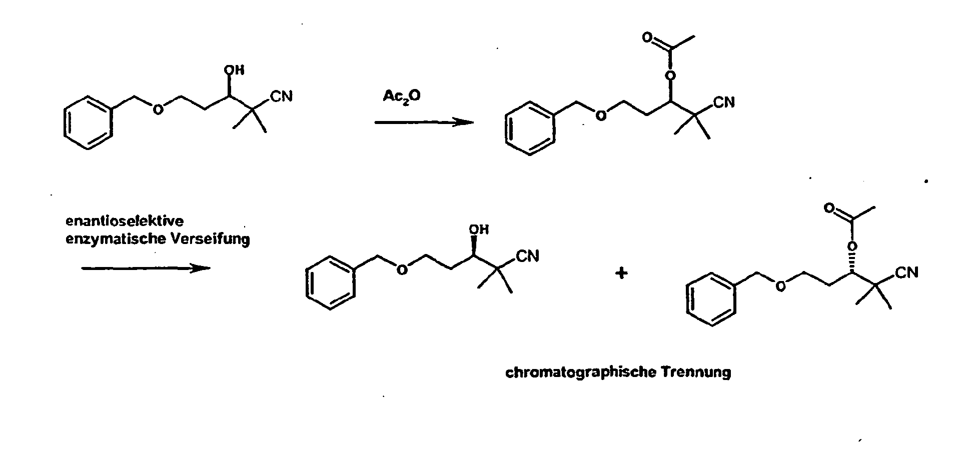
3. Chirale Reduktion eines β-Ketonitils (Noyori-Typ) 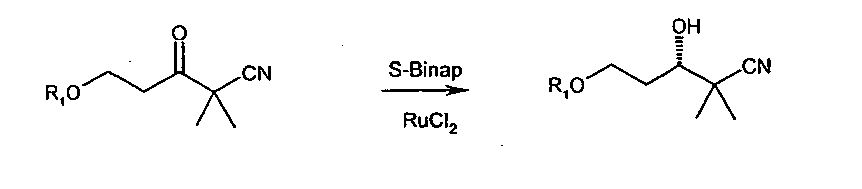
4. Chirale Reduktion des β-Ketoesters mit nachfolgender Reduktion

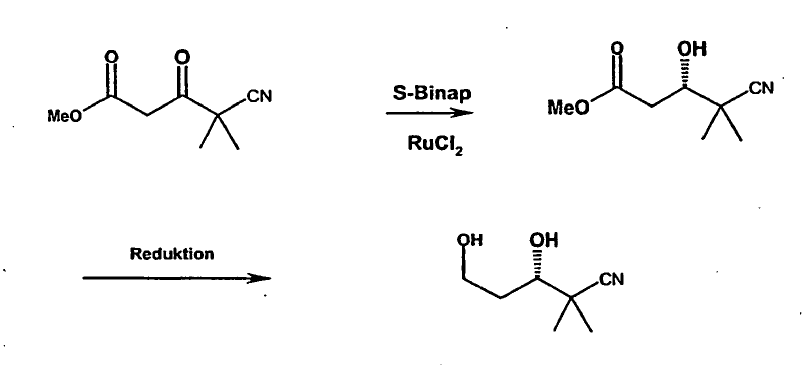

Die Herstellung der erfindungsgemäßen Verbindungen erfolgt bevorzugt auf nachfolgend beschriebenen Sequenzen:
1. Herstellung der Aceton-Ketale 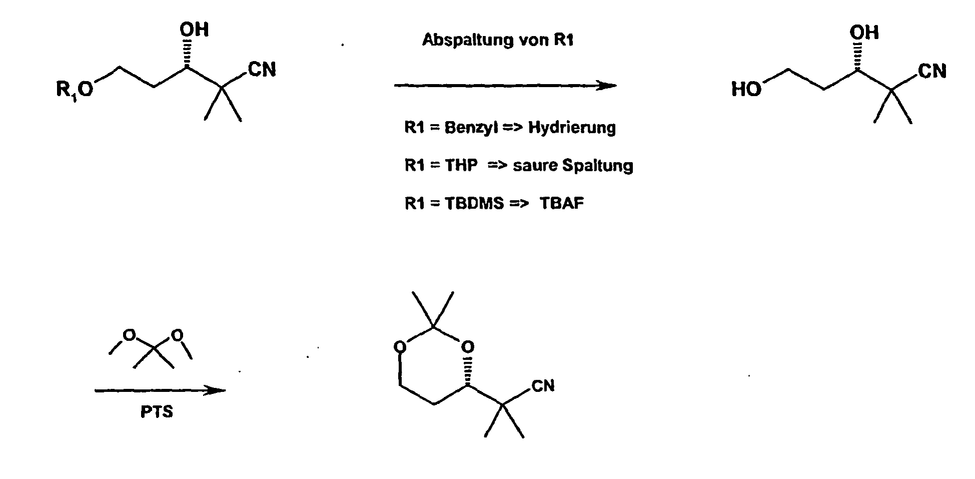
2. Herstellung der Di-TBDMS geschützten Verbindung

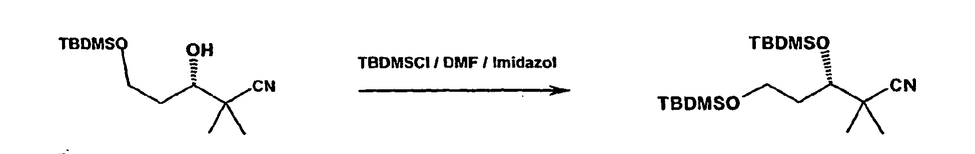

Die Herstellung der erfindungsgemäßen Verbindungen und Verfahren soll nachfolgend an Ausführungsbeispielen näher erläutert werden.

### Beispiel 1:

### Beispiel 1a

### 5-Benzyloxy-2,2-dimethyl-3(R,S)-hydroxy-pentan-nitril

Zu einer LDA-Lösung ( hergestellt aus 33,64g , (79,17 mmol) n-Butyllithium 15% in Hexan, (1,6M) und 80,1 g (79,17 mmol), Diisopropylamin) tropft man bei - 65°C 5,47 g (79,17 mmol) Isobuttersäurenitril und rührt 20 Minuten bei - 65°C. Anschließend wird eine Lösung bestehend aus 10 g ( 60,9 mmol) 3-Benzyloxy-1-propanaldehyd in 20 ml THF zugetropft ( über 60 min ). Die Temperatur wird bei - 65°C gehalten! Dann wird eine Stunde nachgerührt. Nun erwärmt man auf -20°C und tropft eine Lösung aus 20%iger Schwefelsäure zu und lässt die Temperatur auf + 10°C kommen. Dann wird 50 ml MTB-Ether zugegeben und anschließend die organische Phase abgetrennt. Die organische Phase wird mit Wasser und anschließend mit gesättigter Natriumhydrogencarbonat gewaschen. Zum Schluss wird noch einmal mit Wasser gewaschen und dann im Vakuum zur Trockene eingeengt.
Ausbeute: 13,1 g ( 92% d.Th. ) farbloses Öl.

**Elementaranalyse :**

| | C | H | N |
|---|---|---|---|
| Ber. | 72,07 | 8,21 | 6,00 |
| Gef. | 72,34 | 8,43 | 5,85 |

### Beispiel 1b

### 5-Benzoxy-2,2-dimethyl-3(R,S)-acetoxy-pentan-nitril

Zu einer Lösung aus 25,6 g (109,7 mmol) 5-Benzyloxy-2,2-dimethyl-3-hydroxypentan-nitril der Titelverbindung aus Beispiel **1a,** 14,43 g ( 142,64 mmol) Triethylamin und 200 mg 4-Dimethylaminopyridin (DMAP), gelöst in 128 ml MTB-Ether gibt man bei 0°C 14,56 g (42,64 mmol) Essigsäureanhydrid zu und rührt 5 Stunden bei Raumtemperatur. Man gießt die Reaktionsmischung auf 2 I Eiswasser und extrahiert 2 mal mit je 300 ml MTB-Ether. Die vereinigten MTB- Phasen werden einmal mit 300 ml 5%iger Salzsäure und anschließend mit Wasser gewaschen. Man dampft im Vakuum zur Trockene ein.
Ausbeute : 28,82 g (95% d.Th.) , farbloses Öl.

**Elementaranalyse :**

| | C | H | N |
|---|---|---|---|
| Ber. | 69,79 | 7,69 | 5,09 |
| Gef. | 69,51 | 8,01 | 4,83 |

### Beispiel 1c

### 5-Benzyloxy-2,2-dimethyl-3(S)-hydroxy-pentan-nitril

10 g (36,31 mmol) 5-Benzyloxy-2,2-dimethyl-3(R,S)-acetoxy-pentan-nitril der Titelverbindung aus Beispiel **1b** werden in eine Pufferlösung, hergestellt aus 0,88 Kaliumdihydrogenphosphat und 1,82 g Dinatriumhydrogenphosphat in 250 ml Wasser gegeben. Anschließend setzt man 5 g des Enzyms Lipase AYS"Amano" (bezogen von Amano) zu und rührt 24 Stunden bei 40°C. Der pH-Wert wird durch Zugabe von 2,062 g Dinatriumhydrogenphosphat auf pH = 7 gebracht und anschließend unter HPLC-Verfolgung im Abstand von 12 Stunden unter HPLC Verfolgung solange weitergerührt, bis der Peak des R-Acetates kleiner als 1 Flächen-% ist..Aufarbeitung: Man extrahiert 2 mal mit 200 ml Ethylacetat. Die organischen Phasen werden vereinigt und im Vakuum zur Trockene eingedampft. Die Reinigung erfolgt durch Chromatographie an Kieselgel (Hexan/Ethylacetat-Gradient). Es werden mit der 1 Fraktion 4.2 g (45% der Theorie) 5-Benzyloxy-2,2-dimethyl-3(**R**)-hydroxy-pentan-nitril und mit der 2. Fraktion 4.8 g (48 % d. Theorie) 5-Benzyloxy-2,2-dimethyl-3(**S**)-acetoxy-pentan-nitril erhalten.

4.8 g (17,5 mmol) 5-Benzyloxy-2,2-dimethyl-3(**S**)-acetoxy-pentan-nitril aus der 2. Fraktion werden in 50 ml Methanol gelöst und mit 1,4 g (35 mmol) NaOH versetzt. Es wird 3 h bei 25°C gerührt, auf 200 ml Wasser gegeben, mit 2 x 200 ml MTB Ether extrahiert, über Natriumsulfat getrocknet und eingeengt.

Ausbeute: 4 g ( 47 % d.Th. ) 5-Benzyloxy-2,2-dimethyl-3(**S**)-hydroxy-pentan-nitril als farbloses Öl.

**Elementaranalyse :**

| | C | H | N |
|---|---|---|---|
| Ber. | 72,07 | 8,21 | 6,00 |
| Gef. | 71,85 | 8,41 | 5,87 |

### Beispiel 1d

### 5-Hydroxy-2,2-dimethyl-3(S)-hydroxy-pentan-nitril

Zu 11,13 g (47,70 mmol) 5-Benzyloxy-2,2-dimethyl-3(**S**)-hydroxy-pentan-nitril der Titelverbindung aus Beispiel **1c,** gelöst in 110 ml Tetrahydrofuran gibt man 16 g Pearlman-Katalysator (Pd(OH)₂ auf Kohle, 20%). Nun hydriert man 7,5 Stunden bei 10 bar und Raumtemperatur . Es wird vom Katalysator abfiltriert und das Filtrat im Vakuum zur Trockene eingeengt.
Ausbeute: 6,73 g (98 % d,Th,) eines farblosen zähen Öls.

**Elementaranalyse**

| | C | H | N |
|---|---|---|---|
| Ber. | 58,72 | 9,15 | 9,78 |
| Gef. | 58,64 | 9,23 | 9,69 |

### Beispiel 1e

### 3(S)-(3,5 ) Acetondimethylketal -2,2-dimethyl -pentan-nitril

6,73 g ( 47 mmol) 5-Hydroxy-2,2-dimethyl-3(**S**)-hydroxy-pentan-nitril der Titelverbindung aus Beispiel **1d** werden in 27 ml Acetondimethylketal gelöst und 546 mg Campher-10-Sulfonsäure zugegeben. Man erhitzt 15 Stunden auf 50°C. Man engt im Vakuum zur Trockene ein. Nimmt den Rückstand in 200 ml Methylenchlorid auf und wäscht mit gesättigter Natriumhydrogencarbonat-Lösung, anschließend mit gesättigter Natriumchlorid-Lösung. Die organische Phase wird über Natriumsulfat getrocknet und im Vakuum zur Trockene eingeengt. Das erhaltene Öl kristallisiert beim Stehen.
Ausbeute: 5,55 g , (77 % d,Th,) farbloser kristalliner Feststoff .

**Elementaranalyse :**

| | C | H | N |
|---|---|---|---|
| Ber. | 65,54 | 9,35 | 7,64 |
| Gef. | 65,38 | 9,29 | 7,58 |

### Beispiel 2

### 3(S)- 3,5-Di-tert.-butyldimethylsilytoxy-2,2-dimethyl -pentan-nitril

Zu einer Lösung aus 3 g (20,95 mmol) 5-Hydroxy-2,2-dimethyl-3(**S**)-hydroxy-pentan-nitril der Titelverbindung aus Beispiel **1d,** in 20 ml Dimethylformamid gelöst, gibt man 7,13g (104,75 mmol) Imidazol und 7,9 g (52,37 mmol) tert-Butyldimethylsilylchlorid und rührt 16 Stunden bei Raumtemperatur. Die Lösung gießt man auf 200 ml Wasser und extrahiert 2 mal mit je 50 ml Cyclohexan . Die organischen Phasen werden vereinigt und im Vakuum zur Trockene eingedampft. Der Rückstand wird über Flash-Chromatographie an Kieselgel (Hexan / MTB-Ether) gereinigt .
Ausbeute: 7,39 g , ( 95 % d,Th,) eines farblosen zähen Öl.

**Elementaranalyse :**

| | C | H | N |
|---|---|---|---|
| Ber. | 61,39 | 11,12 | 3,77 |
| Gef. | 62,00 | 11,30 | 3,80 |

### Beispiel 3

### 3(S)- 3,5-Cyclohexanonketal-2,2-dimethyl -pentan-nitril

Zu einer Lösung aus 3 g (20,95 mmol) 5-Hydroxy-2,2-dimethyl-3(**S**)-hydroxy-pentan-nitril der Titelverbindung aus Beispiel **1f** in 30,21 g (0,2095 mol Cyclohexanondimethylketal gibt man 10 mg p-Toluolsulfonsäure und rührt 6 Stunden bei 100°C. Die Lösung gießt man auf 200 ml Wasser und extrahiert 2 mal mit je 50 ml Ethylacetat . Die organische Phasen werden vereinigt und im Vakuum zur Trockene eingedampft. Der Rückstand wird über Flash-Chromatographie an Kieselgel (Hexan / MTB-Ether) gereinigt.
Ausbeute: 4,21 g (90 % d.Th.) eines farblosen zähen Öls.

**Elementaranalyse :**

| | C | H | N |
|---|---|---|---|
| Ber. | 69,92 | 9,48 | 6,27 |
| Gef. | 69,81 | 9,62 | 6,15 |

### Beispiel 4

### 3(S)-3,5-Benzaldehydacetal-2,2-dimethyl -pentan-nitril

Zu einer Lösung aus 3 g (20,95 mmol) 5-Hydroxy-2,2-dimethyl-3(**S**)-hydroxy-pentan-nitril der Titelverbindung aus Beispiel **1f,** in 20 ml Dimethylformamid gelöst, gibt man 31,9 g (0,2095 mol) Benzaldehyd-dimethylacetal und 50 mg p-Toluolsulfonsäure und rührt 16 Stunden bei 100°C. Die Lösung gießt man auf 200 ml Wasser und extrahiert 2 mal mit je 50 ml Ethylacetat. Die organischen Phasen werden vereinigt und im Vakuum zur Trockene eingedampft. Der Rückstand wird über Flash-Chromatographie an Kieselgel (Hexan / MTB-Ether) gereinigt
Ausbeute : 4,26g (88 % d.Th.) eines farblosen zähen Öls.

**Elementaranalyse :**

| | C | H | N |
|---|---|---|---|
| Ber. | 69,92 | 9,48 | 6,27 |
| Gef. | 69,81 | 9,62 | 6,15 |

### Beispiel 5

### 3(S)-3,5-Dichlordiphenylsilan-2,2-dimethyl-pentan-nitril

Zu einer Lösung aus 3 g (20,95 mmol 5-Hydroxy-2,2-dimethyl-3(**S**)hydroxy-pentan-nitril der Titelverbindung aus Beispiel **1f,** in 20 ml Dimethylformamid gelöst, gibt man 3,14 g (46,09 mmol) Imidazol und 5,83 g (23,05 mmol) Dichlordiphenylsilan und rührt 16 Stunden bei Raumtemperatur. Die Lösung gießt man auf 200 ml Wasser und extrahiert 2 mal mit je 50 ml Methylenchlorid. Die organischen Phasen werden vereinigt und im Vakuum zur Trockene eingedampft. Der Rückstand wird über Flash-Chromatographie an Kieselgel (Hexan / MTB-Ether) gereinigt.
Ausbeute : 5,76 g (85 % d.Th.) eines farblosen zähen Öls.

**Elementaranalyse :**

| | C | H | N |
|---|---|---|---|
| Ber. | 70,55 | 6,54 | 4,33 |
| Gef. | 70,41 | 6,71 | 4,25 |

### Beispiel 6a

### 5-Tert-Butyldimethylsilyl-2,2-dimethyl-3(R,S)-hydroxy-pentan-nitril

Zu einer LDA-Lösung (hergestellt aus 28,6 g (66,99 mmol) n-Butyllithium 15%ig (1,6 M) und 6,82 g, 66,99 mmol, Disopropylamin ) tropft man bei - 65°C 4,62 g ( 66,99 mmol) Isobuttersäurenitril und rührt 20 Minuten bei - 65°C. Anschließend wird eine Lösung bestehend aus 11,47 g (60,9 mmol) 5-Tert-Butyldimethylsilyl-1-propanaldehyd in 20 ml THF zugetropft (über 60 min.). Die Temperatur wird bei-65°C gehalten! Dann wird eine Stunde nachgerührt. Nun erwärmt man auf - 20°C und tropft eine Lösung 130 ml 1 N Salzsäure zu und lässt die Temperatur auf + 10°C kommen . Dann wird 50 ml MTB-Ether zugegeben und anschließend die organische Phase abgetrennt. Die organische Phase wird mit Wasser und anschließend gesättigter Natriumhydrogencarbonat-Lösung gewaschen. Zum Schluss wird noch einmal mit Wasser gewaschen und dann im Vakuum zur Trockene eingeengt.
Ausbeute : 13,65 g 87 % d.Th.)

**Elementaranalyse :**

| | C | H | N |
|---|---|---|---|
| Ber. | 60,65 | 10,57 | 5,44 |
| Gef. | 60,48 | 10,65 | 5,37 |

### Beispiel 6b

### 5-Hydroxy-2,2-dimethyl-3(R,S)-hydroxy-pentan-nitril

Zu einer Lösung aus 3 g ( 11,65 mmol) 5-Tert-Butyldimethylsilyl -2,2-dimethyl-3(**R,S**)-hydroxy-pentan-nitril der Titelverbindung aus Beispiel **6a**, in 40 ml Tetrahydrofuran gelöst gibt man 12,18 g (46,61 mmol) Tetrabutylammoniumfluorid-Hydrat und rührt 16 Stunden bei Raumtemperatur. Anschließend wird im Vakuum zur Trockene eingedampft. Der Rückstand wird über RP-18 -Chromatographie (Laufmittel : Acetonitril/ Wasser-Gradient) gereinigt.
Ausbeute: 1,41 g (85 % d.Th.) eines farblosen zähen Öls.

**Elementaranalyse :**

| | C | H | N |
|---|---|---|---|
| Ber. | 58,72 | 9,15 | 9,78 |
| Gef. | 58,51 | 9,23 | 9,64 |

### Beispiel 6c, (-)-Campherketal

### 3(S)-( 3,5 ) Campherdimethylketal -2,2-dimethyl -pentan-nitril

6,73 g (47 mmol 5-Hydroxy-2,2-dimethyl-3(**R,S**)-hydroxy-pentan-nitril der Titelverbindung aus Beispiel **6b** werden in 27 ml Methylenchlorid gelöst mit 93 g (1S)-(-)-Campherketal ( hergestellt aus (1S)-(-)-Campher, Methanol und p-Toluolsulfonsäure ) und 546 mg Campher-10-Sulfonsäure zugegeben. Man erhitzt 15 Stunden am Rückfluss. Man verdünnt den Ansatz in 200 ml Methylenchlorid und wäscht mit gesättigter Natriumhydrogencarbonat-Lösung, anschließend mit gesättigter Natriumchlorid-Lösung. Die organische Phase wird über Natriumsulfat getrocknet und im Vakuum zur Trockene eingeengt. Der Rückstand wird an chiraler Phase chromatographiert (Laufmittel : Acetonitril/ Wasser-Gradient) gereinigt.
Das erhaltene Öl kristallisiert beim Stehen.
Ausbeute : 10 g , (77 % d,Th,) farbloser kristalliner Feststoff.

**Elementaranalysen :**

| | C | H | N |
|---|---|---|---|
| Ber. | 73,61 | 9,81 | 5,05 |
| Gef. | 73,40 | 9,79 | 5,00 |

### Beispiel 6d

### 5-Hydroxy-2,2-dimethyl-3(S)-hydroxy-pentan-nitril

### Spaltung der Campherketals

13 g (47 mmol) 3(**S**)-(3,5) Campherdimethylketal -2,2-dimethyl -pentan-nitril der Verbindung aus Beispiel **6c** werden in 40 ml Tetrahydrofuran gelöst, man gibt 12,18 g (46,61 mmol ) Tetrabutylammoniumfluorid-Hydrat zu und rührt 16 Stunden bei Raumtemperatur; anschließend wird im Vakuum zur Trockene eingedampft. Der Rückstand wird über RP-18 -Chromatographie (Laufmittel : Acetonitril/ Wasser-Gradient) gereinigt.
Ausbeute : 5.72 g (85 % d.Th.) eines farblosen zähen Öls.

**Elementaranalyse :**

| | C | H | N |
|---|---|---|---|
| Ber. | 58,72 | 9,15 | 9,78 |
| Gef. | 58,60 | 9,00 | 9,60 |

### Beispiel 7

### 5-Benzyloxy-2,2-dimethyl-3(S)-hydroxy-pentan-nitril

### und

### 5-Benzyloxy-2,2-dimethyl-3(R)-hydroxy-pentan-nitril

Die Titelverbindung aus Beispiel **1a**, 5-Benzyloxy-2,2-dimethyl-3(**R,S**)-hydroxy-pentan-nitril , wird an chiraler Phase chromatographiert (10 g an Chiralpak AD 20µ/Eluent : Hexan / Ethanol 98:2 Wellenlänge: 208nm)
Man erhält :
**R-Isomer,** Ausbeute : 3,8 g (38 % d.Th.) eines farblosen zähen Öls.

**Elementaranalyse :**

| | C | H | N |
|---|---|---|---|
| Ber. | 58,72 | 9,15 | 9,78 |
| Gef. | 58,59 | 9,31 | 9,71 |

### S-Isomer, Ausbeute : 4,1 g (41% d.Th.) eines farblosen zähen Öls.

**Elementaranalyse :**

| | C | H | N |
|---|---|---|---|
| Ber. | 58,72 | 9,15 | 9,78 |
| Gef. | 58,61 | 9,27 | 9,69 |

### Beispiel 8a

### 5-tert-Butyldimethylsilyl-2,2-dimethyl-3(R,S)acetoxy -pentan-nitril

Zu 28,24 g (109,7 mmol) 5-tert-Butyldimethylsilyl-2,2-dimethyl-3(**R,S**)-hydroxypentan-nitril , der Titelverbindung aus Beispiel **6a,** gibt man14,43 g (142,64 mmol Triethylamin und 200 mg 4-Dimethylaminopyridin (DMAP), gelöst in 128 ml MTB-Ether, bei 0°C 14,56 g (142,64 mmol) Essigsäureanhydrid hinzu und rührt 5 Stunden bei Raumtemperatur . Man gießt auf 2 I Eiswasser und extrahiert 2 mal mit je 300 ml MTB-Ether. Die vereinigten MTB-Phasen werden einmal mit 300 ml 5%iger Salzsäure und anschließend mit Wasser gewaschen. Man dampft im Vakuum zur Trockene ein. Der Rückstand wird über Flash-Chromatographie an Kieselgel ( Hexan / MTB-Ether) gereinigt.
Ausbeute : 31,21 g (95 % d.Th ) farbloses Öl.

**Elementaranalyse :**

| | C | H | N |
|---|---|---|---|
| Ber. | 60,16 | 9,76 | 4,68 |
| Gef. | 60,02 | 9,85 | 4,59 |

### Beispiel 8b

### 5-tert-Butyldimethylsilyl-2,2-dimethyl-3(S)hydroxy -pentan-nitril

10 g (33,39 mmol) 5- tert-Butyldimethylsilyl -2,2-dimethyl-3(**R,S**)-acetoxy-pentan-nitril der Titelverbindung aus Beispiel **8a** werden in eine Pufferlösung, hergestellt aus 0,88 g Kaliumdihydrogenphosphat und 1,82 g Dinatriumhydrogenphosphat in 250ml Wasser gegeben Anschließend setzt man 5 g des Enzyms Lipase AYS"Amano" (bezogen von Amano) zu und rührt 42,5 Stunden bei Raumtemperatur. Der pH-Wert wird durch Zugabe von 2,062 g Dinatriumhydrogenphosphat auf pH = 7 gebracht und anschließend 44,5 Stunden weitergerührt. Aufarbeitung : Man extrahiert 3 mal mit 200 ml Ethylacetat. Die organischen Phasen werden vereinigt und im Vakuum zur Trockene eingedampft. Die Reinigung erfolgt durch Chromatographie an Kieselgel (Hexan/Ethylacetat-Gradient).

Es werden 3.8 g (45%) 5-tert-Butyldimethylsilyl-2,2-dimethyl-3(**R**)-hydroxy -pentan-nitril und 4.8 g (48%) 5- tert-Butyldimethylsilyl -2,2-dimethyl-3(**S**)-acetoxy-pentan-nitril erhalten.
4.8 g (16 mmol) 5- tert-Butyldimethylsilyl -2,2-dimethyl-3(**S**)-acetoxy-pentan-nitril werden in 50 ml Ethanol gelöst und mit 1,28 g NaOH (32 mmol) versetzt. Es wird 3 h bei 25°C gerührt, auf 200 ml Wasser gegeben, mit 2 x 200 ml MTB Ether extrahiert, über Natriumsulfat getrocknet und eingeengt.

Ausbeute : 3,43 g (40 % d.Th.)

**Elementaranalyse :**

| | C | H | N |
|---|---|---|---|
| Ber. | 60,65 | 10,57 | 5,44 |
| Gef. | 60,54 | 10,64 | 5,37 |

### Beispiel 8c

### 3(S)- 3,5-Di-tert.-butytdimethylsilyloxy-2,2-dimethyl -pentan-nitril

Zu einer Lösung aus 3 g ( 11,65 mmol ) 5-tert-Butyldimethylsilyl -2,2-dimethyl-3(**S**)-hydroxy-pentan-nitril der Titelverbindung aus Beispiel **8b**, in 10 ml Dimethylformamid gelöst , gibt man 2,37 g ( 34,95 mmol ) Imidazol und 2,63 g (17,47 mmol) tert.-Butyldimethylsilylchlorid und rührt 16 Stunden bei Raumtemperatur. Die Lösung gießt man auf 100 ml Wasser und extrahiert 2 mal mit je 50 ml MTB-Ether . Die organischen Phasen werden vereinigt und im Vakuum zur Trockene eingedampft.

Der Rückstand wird über Flash-Chromatographie an Kieselgel (Hexan / MTB-Ether) gereinigt.
Ausbeute : 4,11 g (95 % d.Th.) eines farblosen zähen Öls.

**Elementaranalyse :**

| | C | H | N |
|---|---|---|---|
| Ber. | 61,39 | 11,12 | 3,77 |
| Gef. | 61,31 | 11,25 | 3,64 |

### Beispiel 9

### 5-Hydroxy-2,2-dimethyl-3(S)-hydroxy-pentan-nitril

Zu einer Lösung aus 3 g (11,65 mmol) 5-Tert-Butyldimethylsilyl-2,2-dimethyl-3(**S**)hydroxy -pentan-nitril der Titelverbindung aus Beispiel **8b** , in 40 ml Tetrahydrofuran gelöst, gibt man 12,18 g (46,61 mmol Tetrabutylammoniumfluorid-Hydrat und rührt 16 Stunden bei Raumtemperatur. Anschließend wird im Vakuum zur Trockene eingedampft. Der Rückstand wird über RP-18 -Chromatographie (Laufmittel : Acetonitril/ Wasser-Gradient) gereinigt.
Ausbeute : 1,41 g (85 % d.Th.) eines farblosen zähen Öls.

**Elementaranalyse :**

| | C | H | N |
|---|---|---|---|
| Ber. | 58,72 | 9,15 | 9,78 |
| Gef. | 58,61 | 9,23 | 9,69 |

### Beispiel 10a

### 5-Benzyloxy-2,2-dimethyl-3-keto-pentan-nitril

Zu einer LDA-Lösung ( hergestellt aus 33,64 g (79,17 mmol) n-Butyllithium (15%ig. , 1.6M) und 80,1 g (79,17 mmol) Diisopropylamin ) tropft man bei - 65°C 5,47 g ( 79,17 mmol ) Isobuttersäurenitril und rührt 20 Minuten bei - 65°C. Anschließend wird eine Lösung bestehend aus 14,29 g ( 71,97 mmol) 3-Benzyloxy-1-propionsäurechlorid in 20 ml THF zugetropft (60 min.). Die Temperatur wird bei - 65°C gehalten! Dann wird eine Stunde nachgerührt . Man erwärmt auf - 20°C und tropft eine Lösung aus 20%iger Schwefelsäure zu und lässt die Temperatur auf + 10°C kommen. Dann wird 50 ml MTB-Ether zugegeben und anschließend die organische Phase abgetrennt. Die organische Phase wird mit Wasser und anschließend mit gesättigter Natriumhydrogencarbonat-Lösung gewaschen . Zum Schluss wird noch einmal mit Wasser gewaschen und dann im Vakuum zur Trockene eingeengt. Der Rückstand wird über Flash-Chromatographie an Kieselgel (Hexan / MTB-Ether) gereinigt.
Ausbeute : 14,15 g (85% d.Th.) eines farblosen zähen Öls.

**Elementaranalyse :**

| | C | H | N | O |
|---|---|---|---|---|
| Ber. | 72,70 | 7,41 | 6,06 | 13,83 |
| Gef. | 72,54 | 7,58 | 5,87 | |

### Beispiel 10b

### 5-Hydroxy-2,2-dimethyl-3-keto-pentan-nitril

Zu 10 g (43,23 mmol) 5-Benzyloxy-2,2-dimethyl-3-keto -pentan-nitril der Titelverbindung aus Beispiel **10a,** gelöst in 100ml Methanol gibt man 3 g Pearlman-Katalysator (Pd(OH)₂ auf Kohle , 20%). Nun hydriert man 7,5 Stunden bei 10 bar und Raumtemperatur. Es wird vom Katalysator abfiltriert und das Filtrat im Vakuum zur Trockene eingeengt.
Ausbeute : 5,98 g (98% d.Th.) eines farblosen zähen Öls.

**Elementaranalyse :**

| | C | H | N |
|---|---|---|---|
| Ber. | 59,56 | 7,85 | 9,92 |
| Gef. | 59,47 | 7,94 | 9,85 |

### Beispiel 10c

### 3(S),5-Dihydroxy-2,2-dimethyl-pentan-nitril

5 g ( 35,41 mmol ) 5-Hydroxy-2,2-dimethyl-3-Keto -pentan-nitril der Titelverbindung aus Beispiel **10b**, werden mit einem Katalysator (hergestellt aus 233 mg RuCl₂(Ph)₂ und 626 mg R-BINAP nach R.Selke , Angew.Chem. 1998 110, pp.1927-1930), hydriert (bei 40°C und 100 bar). Es wird vom Katalysator abfiltriert und das Filtrat im Vakuum zur Trockene eingeengt.
Ausbeute : 4,96 g (98 % d.Th.) eines farblosen zähen Öls.

**Elementaranalyse :**

| | C | H | N |
|---|---|---|---|
| Ber. | 58,72 | 9,15 | 9,78 |
| Gef. | 58,65 | 9,26 | 9,71 |

### Beispiel 11

### S-3-(2,2-Dimethyl-[1,3]dioxan-4-yl)-3-methyl-butan-2-on

Zu 3,26 g (17,79 mmol) der Titelverbindung aus Beispiel **1e**, 3(**S**)-( 3,5) Acetondimethylketal -2,2-dimethyl -pentan-nitril, gelöst in 5 ml Diethylether werden bei - 20°C 35,6 ml Methyllithium-Lithiumbromid-Komplex (1,5 M in Diethylether) getropft. Anschließend wird 30 min. bei - 20°C gerührt und dann auf Raumtemperatur erwärmt. Man rührt über Nacht bei Raumtemperatur. Man gibt 10 ml gesättigte Ammoniumchlorid-Lösung zu und rührt 6 Stunden bei Raumtemperatur. Die organische Phase wird abgetrennt und 2 mal mit Wasser gewaschen. Die organische Phase wird im Vakuum zur Trockene eingeengt. Die Reinigung erfolgt durch Chromatographie an Kieselgel ( Hexan/Ethylacetat-Gradient ).
Ausbeute : 2,77 g (78 % d.Th.) eines Öls.

**Elementaranalyse :**

| | C | H |
|---|---|---|
| Ber. | 65,97 | 10,07 |
| Gef. | 65,84 | 10,19 |

### Beispiel 12

### S-1,3-Bis-(tert-butyl-dimethyl-silanyloxy)-4,4-dimethyl-pentan-5-on

Zu 5 g (13,45 mmol) der Titelverbindung aus Beispiel **2**, 3(**S**)- 3,5-Di-tert.-butyldimethylsilyloxy-2,2-dimethyl -pentan-nitril, gelöst in 5 ml Diethylether werden bei - 20°C 40,35 ml Llithiumethylat (1 M Lsg. in THF) getropft. Anschließend wird 30 min. bei - 20°C gerührt und dann auf Raumtemperatur erwärmt. Man rührt über Nacht bei Raumtemperatur. Man gibt 10 ml gesättigte Ammoniumchlorid-Lösung zu und rührt 6 Stunden bei Raumtemperatur. Die organische Phase wird abgetrennt und 2 mal mit Wasser gewaschen. Die organische Phase wird im Vakuum zur Trockene eingeengt. Die Reinigung erfolgt durch Chromatographie an Kieselgel (Hexan/Ethylacetat-Gradient) .
Ausbeute : 4,06 g (75% d.Th.) eines Öls

**Elementaranalyse :**

| | C | H |
|---|---|---|
| Ber. | 62,63 | 11,51 |
| Gef. | 62,51 | 11,64 |

### Beispiel 13

### S-2-(2,2-Dimethyl-[1,3]dioxan-4-yl)-2-methyl-heptan-3-on

3,26 g (17,79 mmol) der Titelverbindung aus Beispiel **1e**, 3(**S**)-(3,5) Acetondimethylketal -2,2-dimethyl -pentan-nitril, gelöst in 5 ml THF werden bei - 65°C 34 ml n-Butyllithium 15%ig (1,6 M in Hexan) zugetropft. Anschließend wird 5 Stunden bei - 65°C gerührt und dann auf Raumtemperatur erwärmt. Man rührt über Nacht bei Raumtemperatur. Man gibt 10 ml gesättigte Ammoniumchlorid-Lösung zu und rührt 6 Stunden bei Raumtemperatur. Die organische Phase wird abgetrennt und 2 mal mit Wasser gewaschen. Die organische Phase wird im Vakuum zur Trockene eingeengt. Die Reinigung erfolgt durch Chromatographie an Kieselgel (Hexan/Ethylacetat-Gradient) .
Ausbeute : 4,13g (96% d.Th.) eines Öls

**Elementaranalyse :**

| | C | H |
|---|---|---|
| Ber. | 69,38 | 10,81 |
| Gef. | 69,27 | 10,96 |

### Beispiel 14

### (4S)-4-(2-Methyl-3-oxo-hept-6-ene-2-yl)-2,2-dimethyl-(1,3)dioxane

Zu 3,26 g (17,79 mmol) der Titelverbindung aus Beispiel **1e ,** 3(**S**)-(3,5) Acetondimethylketal -2,2-dimethyl -pentan-nitril, gelöst in 5 ml Diethylether werden bei - 90°C 50 ml 3-Butenyllithium Lsg. (hergestellt aus 4-Bromo-1-butene und Lithium Draht oder tert-Butyllithium, nach J.Org.Chem, vol 56 No. 21, pp. 6094-6103 (1991) oder J.Chem.Soc. Perkin Trans. I pp. 2937 (1988) ) zugetropft. Anschließend wird 17 Stunden bei - 90°C gerührt und dann auf Raumtemperatur erwärmt . Man rührt über Nacht bei Raumtemperatur 17 Stunden. Man gibt 10 ml gesättigte Ammoniumchlorid-Lösung zu und rührt 6 Stunden bei Raumtemperatur. Die organische Phase wird abgetrennt und 2 mal mit Wasser gewaschen. Die organische Phase wird im Vakuum zur Trockene eingeengt. Die Reinigung erfolgt durch Chromatographie an Kieselgel (Hexan/Ethylacetat-Gradient).
Ausbeute : 2,74g (70 % d.Th.) eines farbloses Öls.

**Elementaranalyse :**

| | C | H |
|---|---|---|
| Ber. | 69,96 | 10,06 |
| Gef. | 69,90 | 10,00 |

### Abkürzungen der verwendeten Ether-Schutzgruppen:

TES = Triethlsilyl
TMS = Trimethylsilyl
TIP = Triisopropyl
TBDPS = tert-Butyl-dimethylsilyl
MEM = Methylethoxymethyl
MOM = Methyloxymethyl
THP = Tetrahydropyranyl-(ether)

## Patentansprüche

1. Verbindungen der allgemeinen Formel I 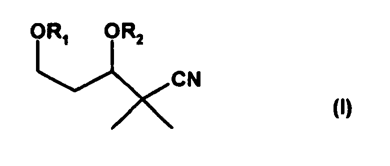 worin
R₁, R₂
gleich oder verschieden sein können und unabhängig voneinander für eine Alkoholschutzgruppe,
oder im Falle wenn R₁ und R₂ verbrückt sind für eine Ketalschutzgruppe stehen.

2. Verbindungen der allgemeinen Formel Ia gemäß Anspruch 1, 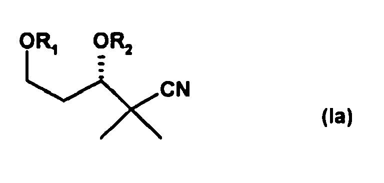 worin
R₁, R₂
gleich oder verschieden sein können und unabhängig voneinander für eine Alkoholschutzgruppe
oder im Falle wenn R₁ und R₂ verbrückt sind für eine Ketalschutzgruppe stehen.

3. Verbindungen der allgemeinen Formel Ib gemäß Anspruch 1, 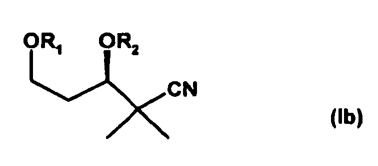 worin
R₁, R₂
gleich oder verschieden sein können und unabhängig voneinander für eine Alkoholschutzgruppe,
oder im Falle wenn R₁ und R₂ verbrückt sind für eine Ketalschutzgruppe stehen.

4. Verbindungen gemäß der Ansprüche 1, 2, 3, **dadurch gekennzeichnet, dass** R₁, R₂ gleich oder verschieden sein können und unabhängig voneinander die Alkoholschutzgruppe Benzyl, 4-Methoxybenzyl , 3,4-Dimethoxybenzyl ,THP, TBDMS, TMS, TES, TIP, TBDPS, MEM , MOM, Allyl, Trityl oder im Falle wenn R₁ und R₂ verbrückt sind, für eine Ketalschutzgruppe 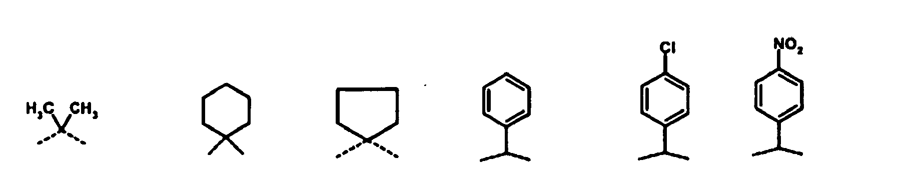 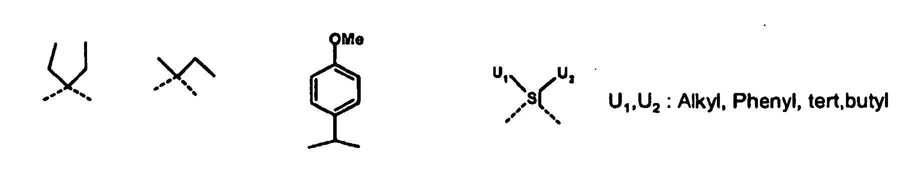 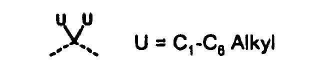 stehen.

5. Verbindungen der Formel Ia gemäß Anspruch 2 ausgewählt aus der Liste
3(**S**)-3,5-Acetondimethylketal-2,2,-dimethyl-pentan-nitril 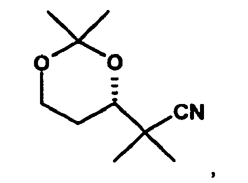 3(**S**)- 3,5-Cyclohexanonketal-2,2,-dimethyl-pentan-nitril 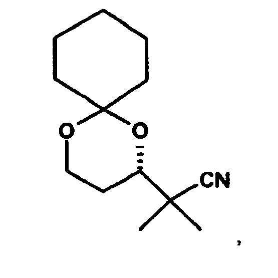 3(**S**)-3,5-Di-tert.-butyldimethylsilyloxy-2,2,-dimethyl-pentan-nitril 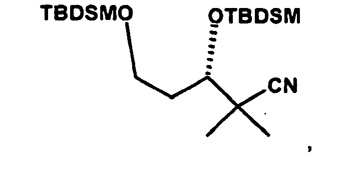 3,5-Benzaldehydacetal-2,2-dimethyl-pentan-nitril 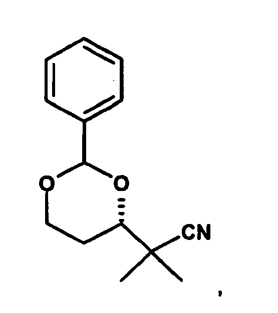 3,5-Diphenylsilan-2,2-dimethyl-pentan-nitril.

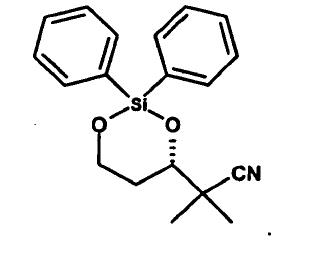

6. Verbindungen der Formel II,
2,2-Dimethyl-3,5-dihydroxy-valeronitril und Isomere

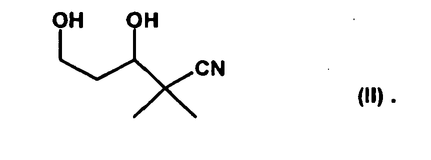

7. Verbindungen gemäß Anspruch 6, **dadurch gekennzeichnet, dass** die Konfiguration am C-Atom des sekundären Alkohol S ist.

8. Verbindungen gemäß Anspruch 6, **dadurch gekennzeichnet, dass** die Konfiguration am C-Atom des sekundären Alkohol R ist.

9. Verbindungen der allgemeinen Formel III 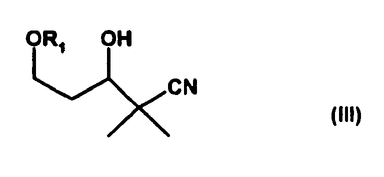 worin
R₁ für eine Alkoholschutzgruppe steht.

10. Verbindungen gemäß Anspruch 9, **dadurch gekennzeichnet, dass** die Alkoholschutzgruppe R₁ für Benzyl, 4-Methoxybenzyl , 3,4-Dimethoxybenzyl, THP, TBDMS, TMS, TES, TIP, TBDPS, MEM, MOM, Allyl, Trityl steht.

11. Verbindungen gemäß Anspruch 9 oder 10, **dadurch gekennzeichnet, dass** die Konfiguration am C-Atom des sekundären Alkohol S ist.

12. Verbindungen gemäß Anspruch 9 oder 10, **dadurch gekennzeichnet, dass** die Konfiguration am C-Atom des sekundären Alkohol R ist.

13. Verbindung gemäß Anspruch 11, 5-Benzyloxy-2,2-dimethyl-3(**S**)-hydroxy-pentan- 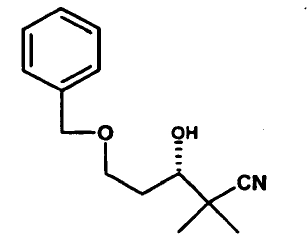 nitril.

14. Verbindungen der allgemeinen Formel XII 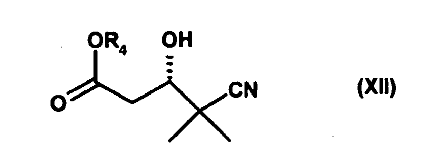 worin
R₄ für eine C₁-C₆ Alkyl, Methyl, Ethyl, tert.Butyl, Phenyl oder Benzylgruppe steht

15. Verbindungen gemäß Anspruch 14, **dadurch gekennzeichnet, dass** die Konfiguration am C-Atom des sekundären Alkohol R ist.

16. Verwendung der Verbindungen nach mindestens einem der Ansprüche 1 bis 15 zur Synthese von natürlichen und synthetischen Epothilonen oder Epothilonderivaten.

17. Verfahren zur Herstellung von Verbindungen gemäß Ansprüchen 1 bis 4, **dadurch gekennzeichnet, dass** man ausgehend von Verbindungen gemäß Anspruch 6 bis 8 die Alkoholgruppen mit den Schutzgruppen R₁ und R₂ schützt.

18. Verfahren gemäß Anspruch 17, **dadurch gekennzeichnet, dass** die Schutzgruppen R₁ und R₂ das Acetonketal und die TBDMS-gruppe darstellen.

19. Verfahren zur Herstellung optisch aktiver Verbindungen der allgemeinen Formel IIIa 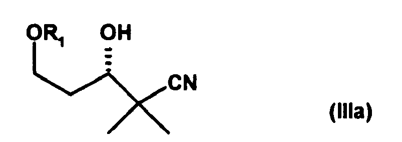 **dadurch gekennzeichnet, dass**
man einen racemischen Ester der allgemeinen Formel VI 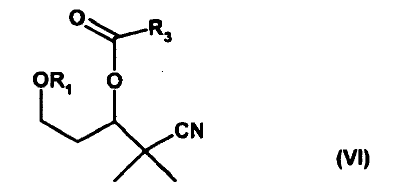 worin
R₁ für eine Alkoholschutzgruppe und
R₃ für ein C₁-C₆ Alkylgruppe oder ein Allyl-, Phenyl-, oder Benzylgruppe
stehen
mittels enzymatischer Verseifung enanantioselektiv verseift.

20. Verfahren gemäß Anspruch 19, **dadurch gekennzeichnet, dass** das zur Verseifung verwendete Enzym Lipase Amano AY ist

21. Verfahren zur Herstellung optisch aktiver Verbindungen der allgemeinen Formel IIIa 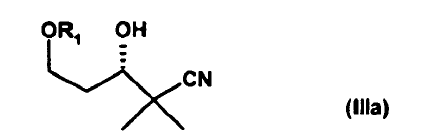 worin
R₁ für eine Alkoholschutzgruppe, **dadurch gekennzeichnet, dass** man ausgehend von Verbindungen der Formel VII 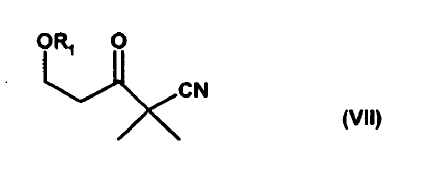 eine chirale Reduktion der Ketogruppe vornimmt.

22. Verfahren gemäß Anspruch 19 oder 21, **dadurch gekennzeichnet, dass** die
Alkoholschutzgruppe R₁ für Benzyl, 4-Methoxybenzyl , 3,4-Dimethoxybenzyl, THP,
TBDMS, TMS, TES, TIP, TBDPS, MEM , MOM, Allyl, Trityl steht.

23. Verfahren gemäß Anspruch 21, **dadurch gekennzeichnet, dass** die chirale Reduktion der Ketogruppe durch katalytische Hydrierung mit einem Noyori-Typ-Katalysator vorgenommen wird.

24. Verfahren gemäß Anspruch 21, **dadurch gekennzeichnet, dass** die chirale Reduktion der Ketogruppe durch enzymatische Reduktion vorgenommen wird.

25. Verfahren zur Herstellung von Verbindungen der allgemeinen Formel XIII 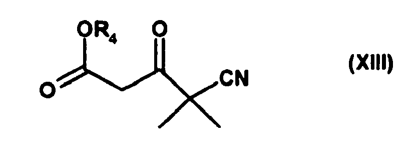 worin
R₄ für eine C₁-C₆ Alkyl, Methyl, Ethyl, tert.Butyl, Phenyl oder Benzylgruppe
steht
**dadurch gekennzeichnet, dass** man Verbindungen der allgemeinen Formel XIV 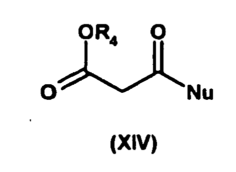 worin
R₄ für eine C₁-C₆ Alkyl, Methyl, Ethyl, tert.Butyl, Phenyl oder Benzylgruppe
Nu für eine Fluchtgruppe wie Cl, Br, Imidazol, -OPh, -O-C₆H₄NO₂, -O-C₁-C₄ Alkyl steht
mit einer Verbindung der Formel V 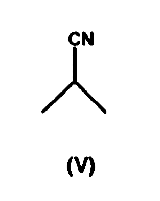 umsetzt.

26. Verfahren zur Herstellung von Ketonen der allgemeinen Formel A 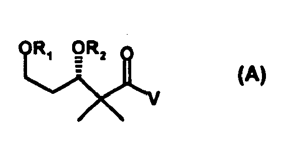 worin R₁,R₂
gleich oder verschieden sein können und unabhängig voneinander für eine Alkoholschutzgruppe
oder im Falle wenn R₁ und R₂ verbrückt sind für eine Ketalschutzgruppe und V für einen C₁-C₅ Alkyl- oder Alkenyl-Rest
steht,
**dadurch gekennzeichnet, dass** man Verbindungen der allgemeinen Formel Ia 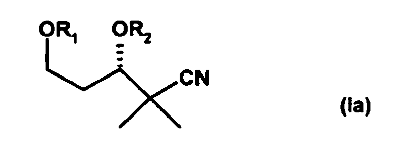 mit Verbindungen der allgemeinen Formel B
M-V (B)
worin M für Li oder MgCl, MgBr, oder MgJ steht
umsetzt
und anschließend unter wässriger Hydrolyse aufarbeitet.

27. Verfahren gemäß Anspruch 26, **dadurch gekennzeichnet, dass** R₁,R₂ gleich oder verschieden sein können und unabhängig voneinander für die Alkoholschutzgruppe Benzyl, 4-Methoxybenzyl , 3,4-Dimethoxybenzyl ,THP, TBDMS, TMS, TES, TIP, TBDPS, MEM , MOM, Allyl, Trityl oder im Falle wenn R₁ und R₂ verbrückt sind, für eine Ketalschutzgruppe 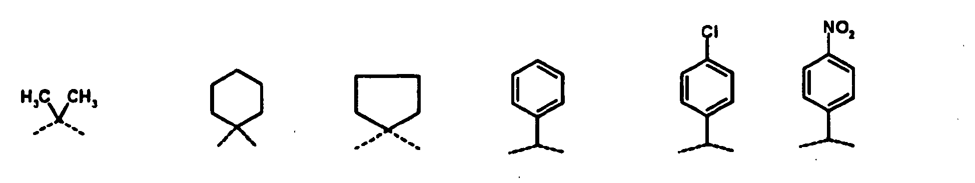 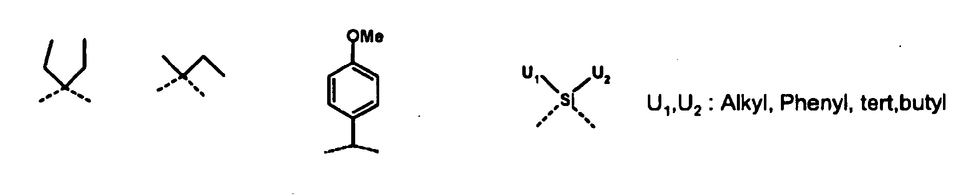 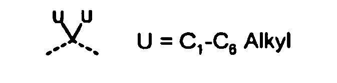 stehen.

28. Verfahren gemäß Anspruch 26 oder 27, **dadurch gekennzeichnet, dass** Verbindungen der allgemeinen Formel M-V für
MeLi, EtLi, Propyl-Li, BuLi, CH₂=CH-CH₂CH₂-Li stehen

## Claims

1. Compounds of general formula I 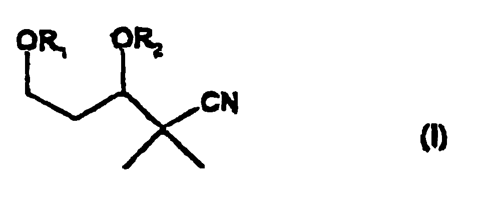 in which
R₁, R₂
can be the same or different and, independently of one another, stand for an alcohol protective group,
or, in the case when R₁ and R₂ are bridged, stand for a ketal protective group.

2. Compounds of general formula Ia according to Claim 1 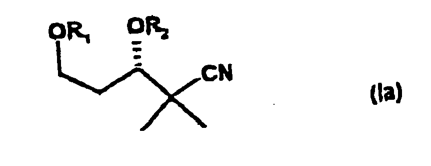 in which
R₁, R2
can be the same or different and, independently of one another, stand for an alcohol protective group,
or, in the case when R₁ and R₂ are bridged, stand for a ketal protective group.

3. Compounds of general formula Ib according to Claim 1 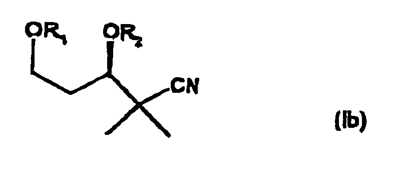 in which
R₁, R₂
can be the same or different and, independently of one another, stand for an alcohol protective group,
or, in the case when R₁ and R₂ are bridged, stand for a ketal protective group.

4. Compounds according to Claims 1, 2, 3,
**characterized in that** R₁, R₂ can be the same or different and, independently of one another, stand for the alcohol protective group, benzyl, 4-methoxybenzyl, 3,4-dimethoxybenzyl, THP, TBDMS, TMS, TES, TIP, TBDPS, MEM, MOM, allyl or trityl, or, in the case when R₁ and R₂ are bridged, stand for a ketal protective group

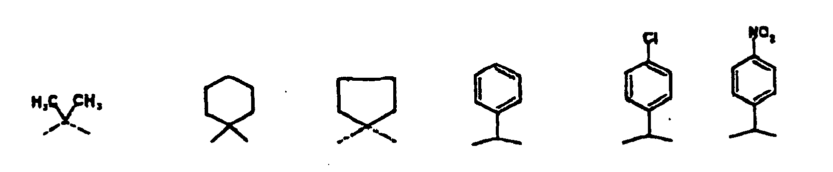

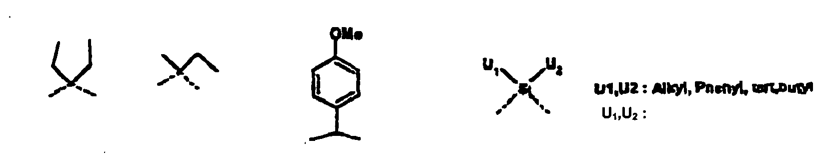

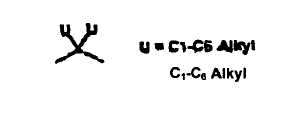

5. Compounds of formula Ia according to Claim 2 selected from the list:
3(S)-3,5-acetone dimethylketal-2,2-dimethyl-pentane-nitrile 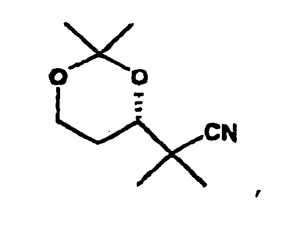 3(S)-3,5-cyclohexanoneketal-2,2-dimethyl-pentane-nitrile 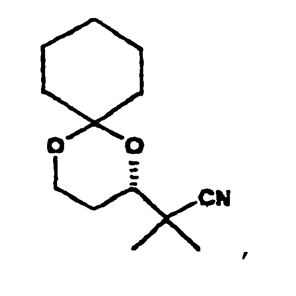 3(S)-3,5-di-tert-butyldimethylsilyloxy-2,2-dimethyl-pentane-nitrile 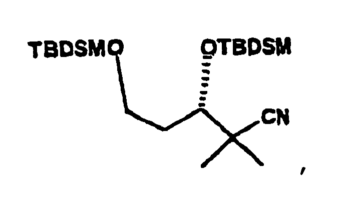 3,5-Benzaldehydeacetal-2,2-dimethyl-pentane-nitrile 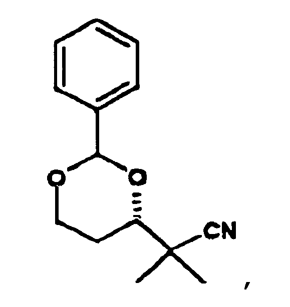 3,5-Diphenylsilane-2,2-dimethyl-pentane-nitrile

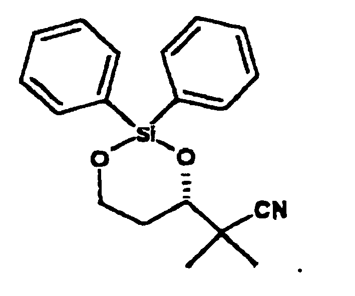

6. Compounds of formula II,
2,2-Dimethyl-3,5-dihydroxy-valeronitrile and isomers

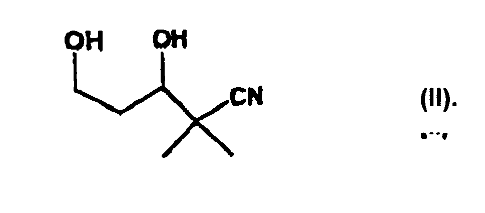

7. Compounds according to Claim 6, **characterized in that** the configuration at the C-atom of the secondary alcohol is S.

8. Compounds according to Claim 6, **characterized in that** the configuration at the C-atom of the secondary alcohol is R.

9. Compounds of general formula III 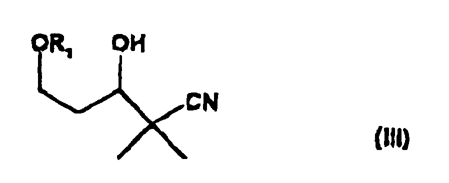 in which
R₁ stands for an alcohol protective group.

10. Compounds according to Claim 9, **characterized in that** the alcohol protective group R₁ stands for benzyl, 4-methoxybenzyl, 3,4-dimethoxybenzyl, THP, TBDMS, TMS, TES, TIP, TBDPS, MEM, MOM, allyl or trityl.

11. Compounds according to Claim 9 or 10,
**characterized in that** the configuration at the C-atom of the secondary alcohol is S.

12. Compounds according to Claim 9 or 10,
**characterized in that** the configuration at the C-atom of the secondary alcohol is R.

13. Compound according to Claim 11, 5-Benzyloxy-2,2-dimethyl-3(S)-hydroxy-pentane-nitrile

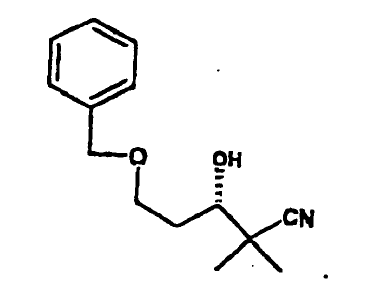

14. Compounds of general formula XII 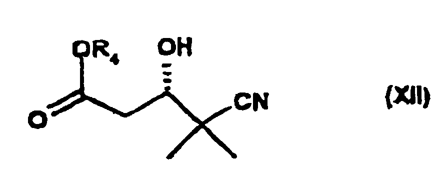 in which
R₄ stands for a C₁-C₆ alkyl, methyl, ethyl, tert-butyl, phenyl or benzyl group.

15. Compounds according to Claim 14, **characterized in that** the configuration at the C-atom of the secondary alcohol is R.

16. Use of the compounds according to at least one of Claims 1 to 15 for the synthesis of natural and synthetic epothilones or epothilone derivatives.

17. Process for the production of compounds according to Claims 1 to 4, **characterized in that** starting from compounds according to Claims 6 to 8, the alcohol groups are protected with protective groups R₁ and R₂.

18. Process according to Claim 17, **characterized in that** the protective groups R₁ and R₂ represent the acetone ketal and the TBDMS group.

19. Process for the production of optically active compounds of general formula IIIa, 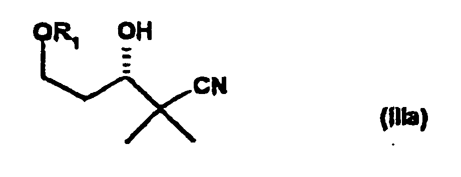 **characterized in that** a racemic ester of general formula VI 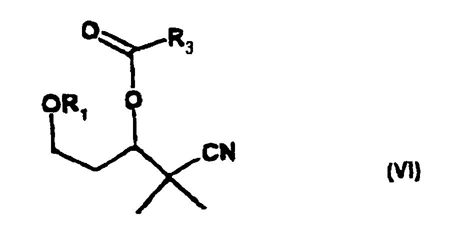 in which
R₁ stands for an alcohol protective group, and
R₃ stands for a C₁-C₆ alkyl group or an allyl, phenyl or benzyl group,
is enantioselectively saponified by means of enzymatic saponification.

20. Process according to Claim 19, **characterized in that** the enzyme that is used for saponification is lipase amano AY.

21. Process for the production of optically active compounds of general formula IIIa 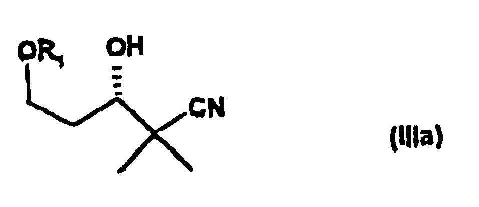 in which
R₁ stands for an alcohol protective group, **characterized in that** starting from compounds of formula VII 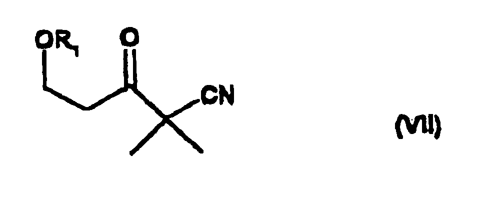 a chiral reduction of the keto group is performed.

22. Process according to Claim 19 or 21, **characterized in that** the alcohol protective group R₁ stands for benzyl, 4-methoxybenzyl, 3,4-dimethoxybenzyl, THP, TBDMS, TMS, TES, TIP, TBDPS, MEM, MOM, allyl or trityl.

23. Process according to Claim 21, **characterized in that** the chiral reduction of the keto group is made by catalytic hydrogenation with a Noyori-type catalyst.

24. Process according to Claim 21, **characterized in that** the chiral reduction of the keto group is performed by enzymatic reduction.

25. Process for the production of compounds of general formula XIII 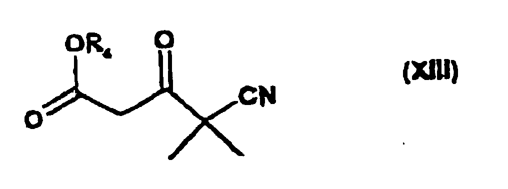 in which
R₄ stands for a C₁-C₆ alkyl, methyl, ethyl, tert-butyl, phenyl or benzyl group, **characterized in that** compounds of general formula XIV 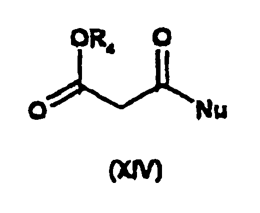 in which
R₄ stands for a C₁-C₆ alkyl, methyl, ethyl, tert-butyl, phenyl or benzyl group, Nu stands for a leaving group, such as Cl, Br, imidazole, -OPh, -O-C₆H₄NO₂, or -O-C₁-C₄ alkyl,
are reacted with a compound of formula V

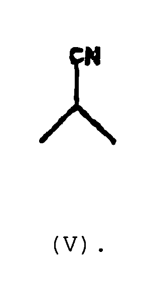

26. Process for the production of ketones of general formula A 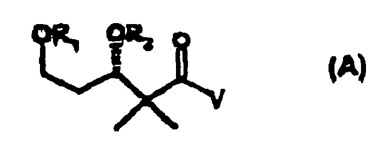 in which R₁, R₂
can be the same or different and, independently of one another, stand for an alcohol protective group,
or, in the case when R₁ and R₂ are bridged, stand for a ketal protective group, and
V stands for a C₁-C₅ alkyl or alkenyl radical,
**characterized in that** compounds of general formula Ia 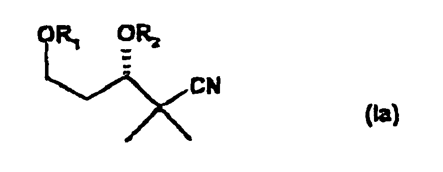 are reacted with compounds of general formula B
M - V (B)
in which M stands for Li or MgCl, MgBr, or MgI, and then are worked up under aqueous hydrolysis.

27. Process according to Claim 26, **characterized in that** R₁, R₂ can be the same or different and, independently of one another the alcohol protective group benzyl, 4-methoxybenzyl, 3,4-dimethoxybenzyl, THP, TBDMS, TMS, TES, TIP, TBDPS, MEM, MOM, allyl or trityl,
or, in the case when R₁ and R₂ are bridged stand for a ketal protective group

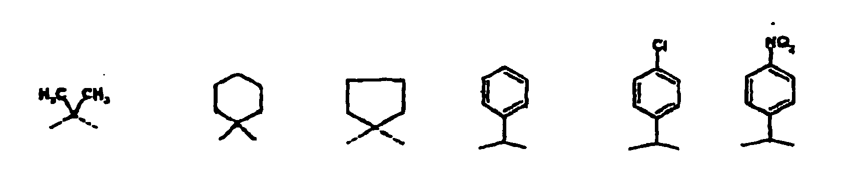

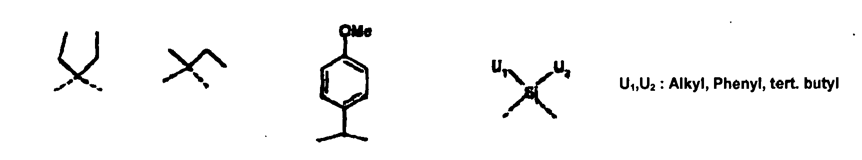

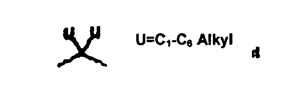

28. Process according to claim 26 or 27, **characterized in that** compounds of general formula M-V stand for MeLi, EtLi, propyl-Li, BuLi, CH₂=CH-CH₂CH₂-Li.

## Revendications

1. Composés de formule générale I 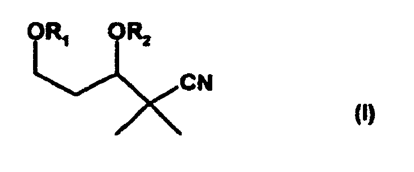 dans laquelle
R₁, R₂
peuvent être identiques ou différents et représentent, indépendamment l'un de l'autre, un groupe protecteur de fonction alcool,
ou, dans le cas où R₁ et R₂ sont pontés, un groupe protecteur cétal.

2. Composés de formule générale Ia selon la revendication 1, 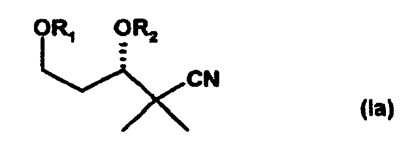 dans laquelle
R₁, R₂
peuvent être identiques ou différents et représentent, indépendamment l'un de l'autre, un groupe protecteur de fonction alcool,
ou, dans le cas où R₁ et R₂ sont pontés, un groupe protecteur cétal.

3. Composés de formule générale Ib selon la revendication 1, 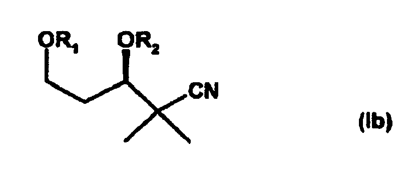 dans laquelle
R₁, R₂
peuvent être identiques ou différents et représentent, indépendamment l'un de l'autre, un groupe protecteur de fonction alcool,
ou, dans le cas où R₁ et R₂ sont pontés, un groupe protecteur cétal.

4. Composés selon les revendications 1, 2, 3,
**caractérisés en ce que** R₁, R₂ peuvent être identiques ou différents et représentent, indépendamment l'un de l'autre, le groupe protecteur de fonction alcool benzyle, 4-méthoxybenzyle, 3,4-diméthoxybenzyle, THP, TBDMS, TMS, TES, TIP, TBDPS, MEM, MOM, allyle, trityle ou, dans le cas où R₁ et R₂ sont pontés, un groupe protecteur cétal

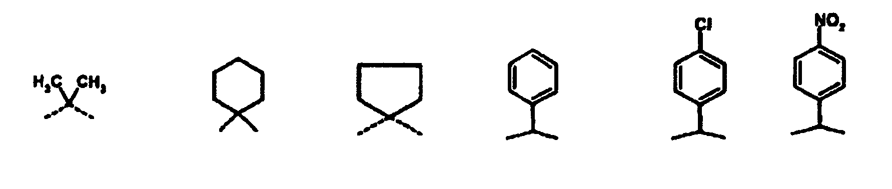

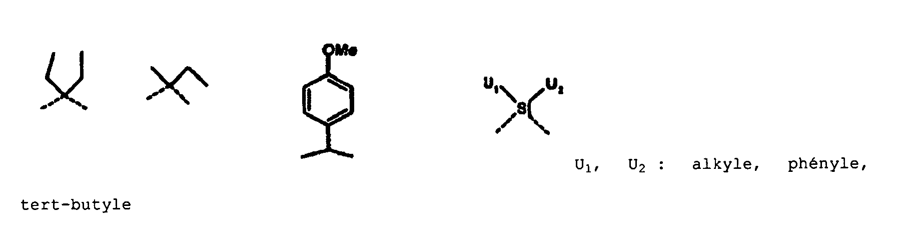

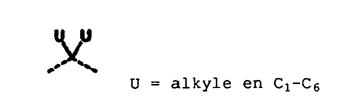

5. Composés de formule Ia selon la revendication 2, choisis dans la liste
3(**S**)-3,5-acétonediméthylcétal-2,2-diméthyl-pentane-nitrile 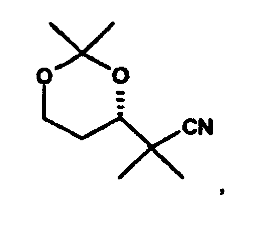 3(**S**)-3,5-cyclohexanonecétal-2,2-diméthyl-pentane-nitrile 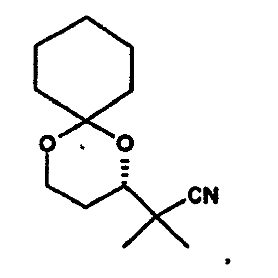 3(**S**)-3,5-di-tert-butyldiméthylsilyloxy-2,2-diméthyl-pentane-nitrile 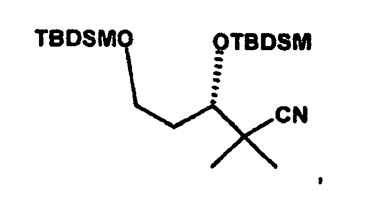 3,5-benzaldéhyde-acétal-2,2-diméthyl-pentane-nitrile 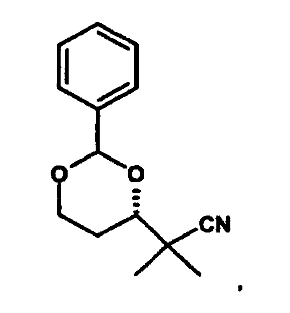 3,5-diphénylsilane-2,2-diméthyl-pentane-nitrile

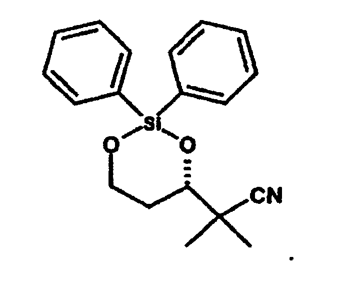

6. Composés de formule II,
2,2-diméthyl-3,5-dihydroxy-valéronitrile et isomères

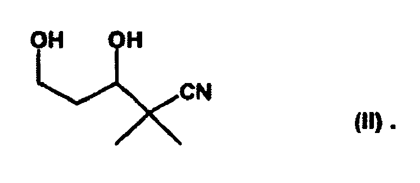

7. Composés selon la revendication 6, **caractérisés en ce que** la configuration au niveau de l'atome de carbone de l'alcool secondaire est S.

8. Composés selon la revendication 6, **caractérisés en ce que** la configuration au niveau de l'atome de carbone de l'alcool secondaire est R.

9. Composés de formule générale III 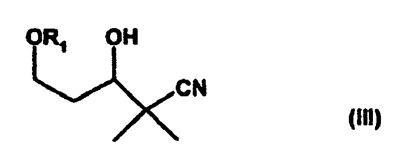 dans laquelle
R₁ représente un groupe protecteur de fonction alcool.

10. Composés selon la revendication 9, **caractérisés en ce que** le groupe protecteur de fonction alcool R₁ représente le groupe benzyle, 4-méthoxybenzyle, 3,4-diméthoxybenzyle, THP, TBDMS, TMS, TES, TIP, TBDPS, MEM, MOM, allyle, trityle.

11. Composés selon la revendication 9 ou 10,
**caractérisés en ce que** la configuration au niveau de l'atome de carbone de l'alcool secondaire est S.

12. Composés selon la revendication 9 ou 10,
**caractérisés en ce que** la configuration au niveau de l'atome de carbone de l'alcool secondaire est R.

13. Composé selon la revendication 11, 5-benzyloxy-
2,2-diméthyl-3(**S**)-hydroxy-pentane-nitrile

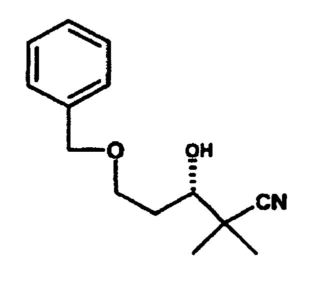

14. Composés de formule générale XII 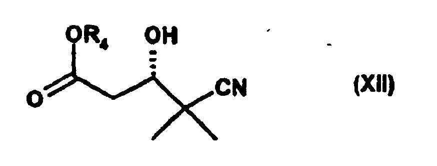 dans laquelle
R₄ représente un groupe alkyle en C₁-C₆, méthyle, éthyle, tert-butyle, phényle ou benzyle.

15. Composés selon la revendication 14, **caractérisés en ce que** la configuration au niveau de l'atome de carbone de l'alcool secondaire est R.

16. Utilisation des composés selon au moins l'une des revendications 1 à 15, pour la synthèse d'épothilones naturelles ou synthétiques, ou de dérivés d'épothilone.

17. Procédé pour la préparation de composés selon les revendications 1 à 4, **caractérisé en ce qu'**à partir de composés selon l'une quelconque des revendications 6 à 8 on protège les groupes alcool par les groupes protecteurs R₁ et R₂.

18. Procédé selon la revendication 17, **caractérisé en ce que** les groupes protecteurs R₁ et R₂ représentent l'acétone-cétal et le groupe TBDMS.

19. Procédé pour la préparation de composés optiquement actifs de formule générale IIIa 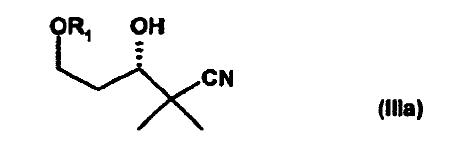 **caractérisé en ce qu'**on saponifie de façon énantiosélective, par saponification enzymatique, un ester racémique de formule générale VI 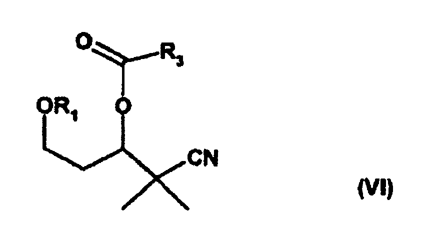 dans laquelle
R₁ représente un groupe protecteur de fonction alcool et
R₃ représente un groupe alkyle en C₁-C₆ ou un groupe allyle, phényle ou benzyle.

20. Procédé selon la revendication 19, **caractérisé en ce que** l'enzyme utilisée pour la saponification est la lipase Amano AY.

21. Procédé pour la préparation de composés optiquement actifs de formule générale IIIa 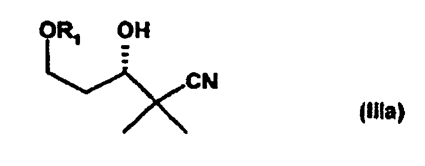 dans laquelle
R₁ représente un groupe protecteur de fonction alcool, **caractérisé en ce qu'**à partir de composés de formule VII 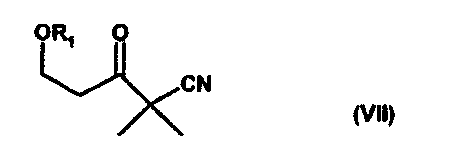 on effectue une réduction chirale du groupe céto.

22. Composés selon la revendication 19 ou 21, **caractérisés en ce que** le groupe protecteur de fonction alcool R₁ représente le groupe benzyle, 4-méthoxybenzyle, 3,4-diméthoxybenzyle, THP, TBDMS, TMS, TES, TIP, TBDPS, MEM, MOM, allyle, trityle.

23. Procédé selon la revendication 21, **caractérisé en ce que** la réduction chirale du groupe céto est effectuée par hydrogénation catalytique à l'aide d'un catalyseur de type Noyori.

24. Procédé selon la revendication 21, **caractérisé en ce que** la réduction chirale du groupe céto est effectuée par réduction enzymatique.

25. Procédé pour la préparation de composés de formule générale XIII 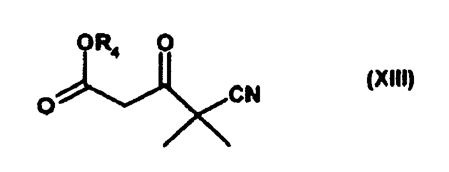 dans laquelle
R₄ représente un groupe alkyle en C₁-C₆, méthyle, éthyle, tert-butyle, phényle ou benzyle,
**caractérisé en ce qu'**on fait réagir des composés de formule générale XIV 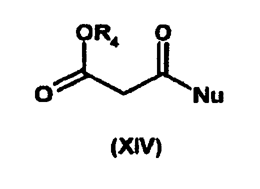 dans laquelle
R₄ représente un groupe alkyle en C₁-C₆, méthyle, éthyle, tert-butyle, phényle ou benzyle,
Nu représente un groupe partant tel que Cl, Br, imidazole, -OPh, -O-C₆H₄NO₂, -O-alkyle(C₁-C₄) avec un composé de formule V

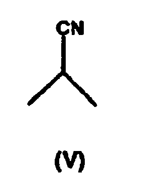

26. Procédé pour la préparation de cétones de formule générale A 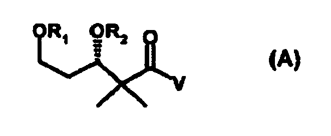 dans laquelle
R₁, R₂ peuvent être identiques ou différents et représentent, indépendamment l'un de l'autre, un groupe protecteur de fonction alcool
ou, dans le cas où R₁ et R₂ sont pontés, un groupe protecteur cétal et
V représente un groupe alkyle ou alcényle en C₁-C₅,
**caractérisé en ce qu'**on fait réagir des composés de formule générale Ia 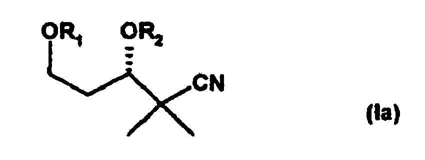 avec des composés de formule générale B
M-V (B)
dans laquelle M représente Li ou MgCl, MgBr ou MgI et ensuite on effectue un traitement final par hydrolyse aqueuse.

27. Procédé selon la revendication 26, **caractérisé en ce que** R₁, R₂ peuvent être identiques ou différents et représentent, indépendamment l'un de l'autre, le groupe protecteur de fonction alcool benzyle, 4-méthoxybenzyle, 3,4-diméthoxybenzyle, THP, TBDMS, TMS, TES, TIP, TBDPS, MEM, MOM, allyle, trityle ou, dans le cas où R₁ et R₂ sont pontés, un groupe protecteur cétal

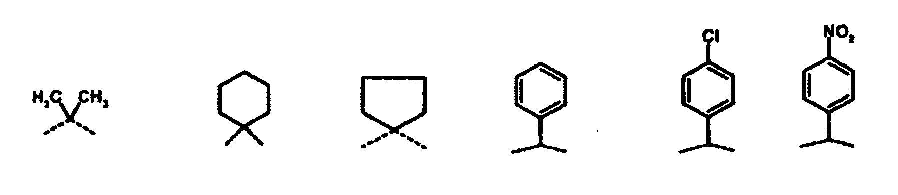

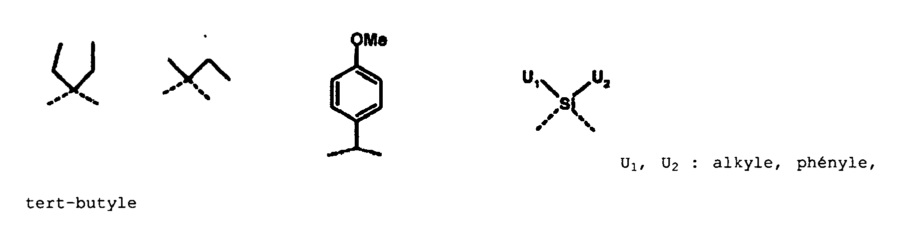

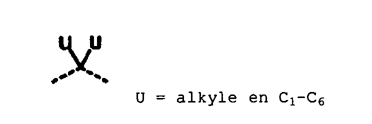

28. Procédé selon la revendication 26 ou 27, **caractérisé en ce que** les composés de formule générale M-V représentent
MeLi, EtLi, propyl-Li, BuLi, CH₂=CH-CH₂CH₂-Li.
